# EUROPÄISCHE PATENTSCHRIFT

(11) **EP 3 613 331 B1**
(45) Veröffentlichungstag und Bekanntmachung des Hinweises auf die Patenterteilung: **26.04.2023**
(21) Anmeldenummer: 19190235.2
(22) Anmeldetag: 06.08.2019
(51) Int. Cl.: A61B 1/00, A61B 1/05, A61B 1/12, G02B 23/24

(54) **ENDOSKOP MIT EINEM BEWEGBAREN BAUTEIL**
ENDOSCOPE WITH A MOVABLE COMPONENT
ENDOSCOPE DOTÉ D'UN COMPOSANT MOBILE

(30) Priorität: 24.08.2018 DE 102018120696
(43) Veröffentlichungstag der Anmeldung: 26.02.2020
(73) Patentinhaber: Karl Storz SE & Co. KG, 78532 Tuttlingen (DE)
(72) Erfinder: Heni, Andreas, 78532 Tuttlingen (DE); Kupferschmid, Markus, 78532 Tuttlingen (DE); Geafer, Lawrence, 78576 Emmingen-Liptingen (DE); Ulmschneider, Daniel, 78532 Tuttlingen (DE)

(56) Entgegenhaltungen:
- EP-A1- 0 774 231
- US-A- 5 381 784
- US-A1- 2006 252 994
- US-A1- 2008 275 298
- US-A1- 2012 232 346
- US-A1- 2014 180 001

## Beschreibung

Die vorliegende Erfindung ist auf ein Endoskop mit einem Schaft und einem bewegbaren Bauteil an einem distalen Ende des Schafts bezogen.

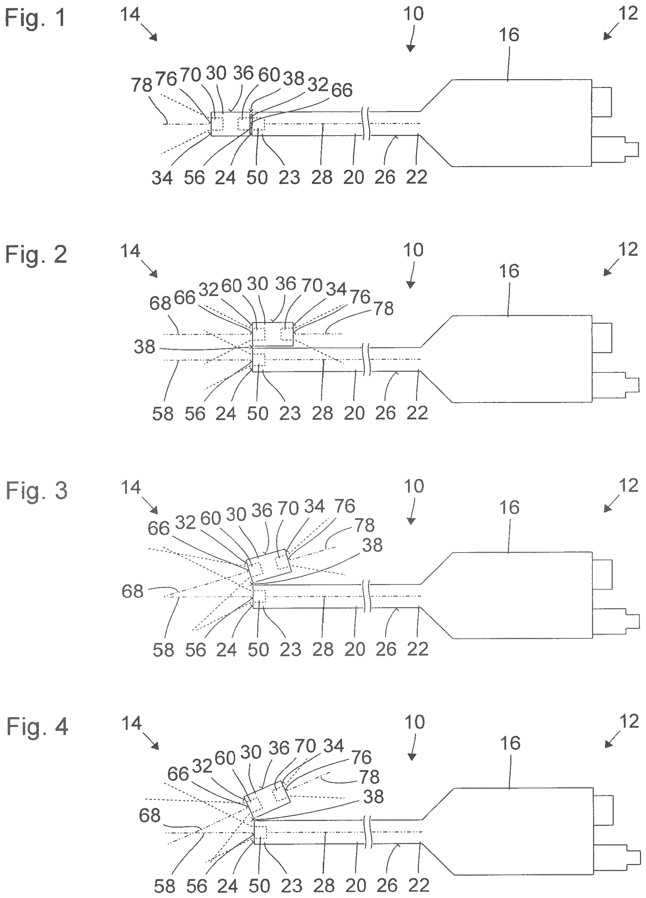

In DE 34 15 771 A1 ist ein Endoskop mit einem flexiblen Schaft beschrieben, dessen distales Ende schwenkbar oder abbiegbar ist (Seite 6, Zeilen 10 bis 13). Ein Gehäuse 26 am proximalen Ende des Endoskops umfasst ein Paar von Zahnstangen 36, 37, die jeweils mit einem Kabel oder Draht 38, 39 verbunden sind (Seite 13, Zeilen 11 bis 22; Figuren 3 bis 5). Mittels einer Steuereinrichtung 29, die einen Hebel 42, in den ein Finger eingreifen kann, oder eine Betätigungsscheibe 45 umfasst, ist ein Zahnrad 41 rotierbar, dessen Rotation die Zahnstangen 36, 37 verschiebt (ebd.).

In DE 39 21 233 C2 ist ein Endoskop mit einer an seinem distalen Ende angeordneten Videoeinheit 1 beschrieben (Titel; Spalte 2, Zeilen 28 bis 43; Spalte 7, Zeilen 30 bis 33; Figur 1). Die Videoeinheit 1 umfasst ein Objektiv 10, einen Bildaufnehmer 11 und eine Beleuchtungseinheit oder eine Lichtaustrittsfläche 7' (Spalte 2, Zeilen 28 bis 43; Spalte 8, Zeilen 10 bis 18; Figur 1). Nach dem Einführen in einen Hohlraum ist die Videoeinheit 1 gegenüber dem distalen Ende des Endoskop-Schafts 2 um eine zur Achse 2' des Endoskop-Schafts 2 parallele und bezogen auf die Stirnfläche der Videoeinheit 1 exzentrische Achse 3 aus der Kontur des Endoskop-Schafts schwenkbar (Spalte 2, Zeilen 28 bis 43; Spalte 8, Zeilen 10 bis 18; Figur 1).

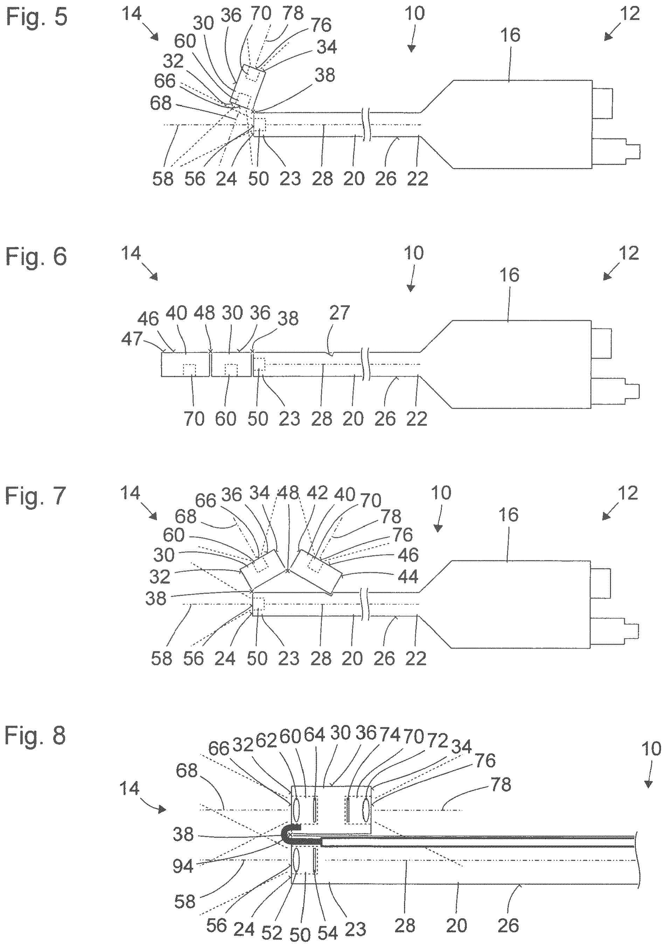

In DE 39 21 233 C2 ist ferner beschrieben, dass an dem distalen Ende des Endoskop-Schafts 2 zwei Videoeinheiten 1', 1" vorgesehen sein können (Spalte 8, Zeilen 62 bis 65). Bewegungselemente 7 beider Videoeinheiten 1, 1' können in unterschiedlichen Kanälen 8', 8" geführt sein (Spalte 9, Zeilen 2 bis 5; Figur 5). Eine der beiden Videoeinheiten 1, 1' kann in Richtung der Längsachse des Endoskop-Schafts verschiebbar sein, so dass beide Videoeinheiten 1, 1' nach dem Ausschwenken in der gleichen Ebene senkrecht zur Endoskop-Längsachse angeordnet sein können, um stereoskopische Aufnahmen zu ermöglichen (Spalte 9, Zeilen 5 bis 12). Eine der beiden Videoeinheiten 1, 1' kann durch eine Lichtquelle oder durch ein Ultraschall-Array oder einen anderen bildgebenden Aufnehmer ersetzt werden (Spalte 9, Zeilen 15 bis 20). Alternativ können beide Videoeinheiten 1, 1' durch zwei Laserdioden oder ein anderes endoskopisches Messsystem ersetzt werden, wobei in einen zentralen Hauptkanal 4 des Endoskops ein herkömmliches optisches System oder eine Videoeinheit eingeführt sein kann (Spalte 9, Zeilen 20 bis 27; Figur 5b).

In DE 39 21 233 C2 ist ferner ein Endoskop mit einer Videoeinheit 1, die um eine zur Achse 2` des Endoskop-Schafts 2 senkrechte Achse 20 schwenkbar ist, beschrieben (Spalte 9, Zeilen 34 bis 37; Figur 6). Die Videoeinheit 1 weist zwei Objektive 10', 10", deren optische Achsen einen 180-Grad-Winkel einschließen, sowie zwei Bildaufnehmer 11', 11" auf (Spalte 9, Zeilen 37 bis 40; Figur 6). In einer Stellung parallel zum Endoskop-Schaft 2 kann die Videoeinheit 1 eine Öffnung eines Kanals 4 im Endoskop-Schaft 2 freigeben (Spalte 9, Zeilen 41 bis 48; Figur 6).

In DE 42 41 938 B4 ist ein Endoskop mit Stereo-Seitblickoptik beschrieben (Titel, Absatz [0001]). In einem abwinkelbaren distalen Bereich des Endoskops sind zwei hintereinander angeordnete Bildaufnehmer jeweils aus einem Bildsensor 5 und einem Objektiv 6, die zur Seite blicken, vorgesehen (Absätze [0023], [0049], Figuren 1 bis 3). Zusätzlich kann ein Bildaufnehmer in Geradeaus-Blick-Richtung vorgesehen sein (Absatz [0060], Figur 4).

In DE 10 2005 045 729 A1 ist ein Beleuchtungssystem für endoskopische Untersuchungen beschrieben (Titel, Zusammenfassung, Absatz [0001]). Eine oder zwei Beleuchtungseinheiten 2 oder Beleuchtungs-Teileinheiten 2a umfassen jeweils mehrere LED-Elemente 3 und sind gegenüber dem distalen Ende eines Endoskopschafts 1 verschwenkbar oder abwinkelbar angeordnet (Absätze [0006], [0007], [0010], [0036], [0043], [0048], [0051], [0056], [0061], Figuren). Zum Schwenken der Beleuchtungseinheiten 2 oder Beleuchtungs-Teileinheiten 2a ist ein im Endoskopschaft 1 gelagertes Betätigungselement, insbesondere eine Zug-/Druckstange oder ein Bowdenzug vorgesehen (Absatz [0047]).

In EP 2 394 567 B1 ist ein Endoskop mit einem Schaft, in dem ein Wärmerohr in Form einer Heatpipe angeordnet ist (Absatz [0001]), um von LEDs erzeugte Wärme abzuführen, beschrieben (Absätze [0003], [0025]).

Eine Vorrichtung gemäß dem Oberbegriff des unabhängigen Anspruchs 1 ist aus der Druckschrift US 5 381 784 A bekannt.

Eine Aufgabe der vorliegenden Erfindung besteht darin, ein verbessertes Endoskop mit einem Schaft und einem bewegbaren Bauteil an einem distalen Ende des Schafts zu schaffen.

Diese Aufgabe wird durch den Gegenstand des unabhängigen Anspruchs gelöst. Weiterbildungen sind in den abhängigen Ansprüchen angegeben.

Ein Endoskop umfasst einen Schaft mit einer Stirnfläche an einem distalen Ende des Schafts, zum Einführen in einen zu betrachtenden Hohlraum, ein bewegbares Bauteil mit einer Stirnfläche, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, eine erste optische Einrichtung an der Stirnfläche des Schafts und eine zweite optische Einrichtung an dem bewegbaren Bauteil, wobei in der ersten Konfiguration die Stirnfläche des bewegbaren Bauteils gegenüber der Stirnfläche des Schafts angeordnet ist oder an dieser anliegt, und wobei in der zweiten Konfiguration das bewegbare Bauteil neben dem distalen Ende des Schafts angeordnet ist.

Das Endoskop ist insbesondere für eine Verwendung bei mikroinvasiven medizinischen Maßnahmen oder für andere medizinische Anwendungen vorgesehen und ausgebildet. Alternativ ist das Endoskop für technische Anwendungen vorgesehen und ausgebildet - ein derartiges Endoskop wird oft auch als Boroskop bezeichnet. Der Schaft des Endoskops kann vollständig gerade oder teilweise oder vollständig gekrümmt, vollständig starr oder teilweise oder vollständig flexibel ausgebildet sein.

Die Stirnfläche an dem distalen Ende des Schafts kann eben oder gekrümmt sein. Die Stirnfläche ist insbesondere orthogonal oder im Wesentlichen orthogonal zu der Längsachse des Schafts angeordnet, d. h. der Winkel zwischen der Flächennormalen oder der mittleren Flächennormalen der Stirnfläche oder der Flächennormalen der Stirnfläche in deren Mitte einerseits und der Längsachse des Schafts andererseits beträgt nicht mehr als 5 Grad oder nicht mehr als 10 Grad. Alternativ kann die Stirnfläche an dem distalen Ende des Schafts geneigt sein.

In der ersten Konfiguration des Endoskops ist das bewegbare Bauteil insbesondere so angeordnet, dass es den Schaft nach distal verlängert. In dieser ersten Konfiguration kann der Schaft mit dem bewegbaren Bauteil voran beispielsweise durch eine Trokarhülse oder durch einen anderen engen Zugang in einen Hohlraum eingeführt werden.

In der ersten Konfiguration können die Stirnfläche des bewegbaren Bauteils und die Stirnfläche am distalen Ende des Schafts - insbesondere wenn sie jeweils eben oder im Wesentlichen eben ausgebildet sind - parallel oder im Wesentlichen parallel angeordnet sein. Dabei kann die Stirnfläche des bewegbaren Bauteils die Stirnfläche an dem distalen Ende des Schafts punkt- oder linienförmig oder flächig berühren.

In der zweiten Konfiguration des Endoskops ist das bewegbare Bauteil teilweise oder vollständig neben dem distalen Ende des Schafts angeordnet. Dabei ist die Stirnfläche an dem bewegbaren Bauteil insbesondere neben oder im Wesentlichen neben der Stirnfläche an dem distalen Ende des Schafts angeordnet und im Wesentlichen in die gleiche Richtung orientiert.

In dieser zweiten Konfiguration könnte der Schaft mit dem bewegbaren Bauteil nur durch eine Öffnung mit einem deutlich vergrößerten Querschnitt eingeführt werden. Diese zweite Konfiguration kann jedoch die Erfassung eines Stereobilds oder eines vergrößerten Raumwinkelbereichs mittels mehrerer analoger (insbesondere rein optischer) oder digitaler Bilderfassungseinrichtungen ermöglichen oder vereinfachen oder andere Vorteile für die Funktionalität des Endoskops aufweisen.

Die erste optische Einrichtung an der Stirnfläche des Schafts ist insbesondere unmittelbar proximal der Stirnfläche an dem distalen Ende des Schafts und an diese Stirnfläche angrenzend angeordnet. Die erste optische Einrichtung ist insbesondere dauerhaft und nicht zerstörungsfrei lösbar mit dem distalen Ende des Schafts verbunden oder in dieses integriert.

Die erste optische Einrichtung umfasst insbesondere ein Objektiv zum Erzeugen eines reellen Bilds, das mittels einer Relaislinsenanordnung oder eines geordneten Bündels von Lichtleitfasern oder auf andere Weise nach proximal übertragen und/oder durch einen oder mehrere Bildsensoren erfasst werden kann. Alternativ oder zusätzlich kann die erste optische Einrichtung an dem distalen Ende des Schafts eine oder mehrere Lichtaustrittsflächen von einem oder mehreren Lichtleitern zum Übertragen von Beleuchtungslicht und/oder eine oder mehrere Leuchtdioden oder andere Lichtquellen zum Erzeugen von Beleuchtungslicht umfassen.

Die zweite optische Einrichtung ist an, insbesondere in dem bewegbaren Bauteil angeordnet. Die zweite optische Einrichtung ist insbesondere dauerhaft und nicht zerstörungsfrei lösbar mit dem bewegbaren Bauteil verbunden oder in dieses integriert.

Die zweite optische Einrichtung umfasst insbesondere ein Objektiv oder eine andere Einrichtung zum Erzeugen eines reellen Bilds und einen oder mehrere Bildsensoren zum Erfassen dieses Bilds. Alternativ oder zusätzlich kann die zweite optische Einrichtung eine oder mehrere Lichtaustrittsflächen von einem oder mehreren Lichtleitern zum Übertragen von Beleuchtungslicht und/oder eine oder mehrere Leuchtdioden oder andere Lichtquellen zum Erzeugen von Beleuchtungslicht umfassen.

In Gegensatz zu dem in DE 39 21 233 C2 anhand der Figur 6 beschriebenen Endoskop kann das hier beschriebene Endoskop beispielsweise die Erfassung eines Stereobilds ermöglichen, wobei ein Teilbild des Stereobilds durch die erste optische Einrichtung und ein weiteres Teilbild durch die zweite optische Einrichtung erzeugt wird. Ferner kann bei dem hier beschriebenen Endoskop die erste optische Einrichtung ein reelles Bild erzeugen, das von einem Relaislinsensystem oder einem geordneten Bündel von Lichtleitfasern oder einer anderen Bildübertragungseinrichtung entlang des Schafts bis zu dem proximalen Ende des Endoskops übertragen werden kann.

Bei einem Endoskop, wie es hier beschrieben ist, ist das bewegbare Bauteil insbesondere um eine vorbestimmte Schwenkachse orthogonal zu einer Längsachse des Schafts relativ zu dem Schaft schwenkbar.

Bei einem Endoskop, wie es hier beschrieben ist, ist das bewegbare Bauteil insbesondere um eine vorbestimmte Schwenkachse orthogonal zu einer Längsachse des Schafts schwenkbar, wobei die vorbestimmte Schwenkachse an der Stirnfläche des Schafts angeordnet ist.

Ein Endoskop umfasst einen Schaft mit einer Stirnfläche an einem distalen Ende des Schafts, zum Einführen in einen zu betrachtenden Hohlraum, ein bewegbares Bauteil mit einer Stirnfläche, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, wobei in der ersten Konfiguration die Stirnfläche des bewegbaren Bauteils gegenüber der Stirnfläche des Schafts angeordnet ist oder an dieser anliegt, wobei in der zweiten Konfiguration das bewegbare Bauteil neben dem distalen Ende des Schafts angeordnet ist, wobei das bewegbare Bauteil um eine vorbestimmte Schwenkachse orthogonal zu einer Längsachse des Schafts schwenkbar ist, und wobei die vorbestimmte Schwenkachse an der Stirnfläche des Schafts angeordnet ist.

Die vorbestimmte Schwenkachse ist insbesondere an der Stirnfläche des Schafts und an der Stirnfläche des bewegbaren Bauteils angeordnet. Die vorbestimmte Schwenkachse ist insbesondere an oder nahe einem Rand der Stirnfläche des Schafts und an oder nahe dem Rand der Stirnfläche des bewegbaren Bauteils angeordnet.

Die vorbestimmte Schwenkachse ist an einer Stirnfläche angeordnet, wenn der Abstand zwischen der vorbestimmten Schwenkachse und der Stirnfläche nicht größer als die Hälfte oder nicht größer als ein Drittel oder nicht größer als ein Fünftel oder nicht größer als ein Zehntel des Durchmessers des Schafts des Endoskops ist.

Eine Anordnung der vorbestimmten Schwenkachse an der Stirnfläche des Schafts kann eine deutlich robustere Konstruktion ermöglichen, als sie beispielsweise in DE 39 21 233 C2 anhand der Figur 6 dargestellt ist.

Bei einem Endoskop, wie es hier beschrieben ist, ist in der zweiten Konfiguration die Stirnfläche des bewegbaren Bauteils insbesondere neben der Stirnfläche des Schafts angeordnet.

Bei einem Endoskop, wie es hier beschrieben ist, sind in der zweiten Konfiguration die Stirnfläche des bewegbaren Bauteils und die Stirnfläche des Schafts insbesondere im Wesentlichen in einer Ebene angeordnet.

Bei einem Endoskop, wie es hier beschrieben ist, ist die vorbestimmte Schwenkachse insbesondere an dem Rand der Stirnfläche des Schafts und an dem Rand der Stirnfläche des bewegbaren Bauteils angeordnet.

Diese Anordnung kann eine besonders robuste mechanische Verbindung zwischen dem bewegbaren Bauteil und dem distalen Ende des Schafts ermöglichen.

Ein Endoskop, wie es hier beschrieben ist, weist insbesondere keinen Arbeitskanal auf.

Ein Endoskop umfasst einen Schaft mit einer Stirnfläche an einem distalen Ende des Schafts, zum Einführen in einen zu betrachtenden Hohlraum, ein bewegbares Bauteil mit einer Stirnfläche, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, wobei in der ersten Konfiguration die Stirnfläche des bewegbaren Bauteils gegenüber der Stirnfläche des Schafts angeordnet ist oder an dieser anliegt, wobei in der zweiten Konfiguration das bewegbare Bauteil neben dem distalen Ende des Schafts angeordnet ist, und wobei das Endoskop keinen Arbeitskanal aufweist.

Ein Arbeitskanal ist ein teilweise oder vollständig innerhalb des Querschnitts des Schafts eines Endoskops angeordneter Kanal, durch den in situ ein Instrument zu dem distalen Ende des Schafts geschoben oder ein anderer Gegenstand oder ein Fluid von dem proximalen Bereich des Endoskops zu dem distalen Ende des Schafts oder von dem distalen Ende zu dem proximalen Bereich des Schafts bewegt oder gefördert werden kann.

Der Verzicht auf einen Arbeitskanal schafft Bauraum für die erste optische Einrichtung an dem distalen Ende des Schafts. Dies kann beispielsweise einen größeren Querschnitt, insbesondere eine größere Apertur eines Objektivs der ersten optischen Einrichtung und/oder einen größeren Bildsensor zum Erfassen des von dem Objektiv erzeugten reellen Bilds oder die Anordnung eines Relaislinsensystems oder einer anderen Einrichtung zum Übertragen des von dem Objektiv erzeugten Bilds ermöglichen.

Bei einem Endoskop, wie es hier beschrieben ist, ragt in der ersten Konfiguration das bewegbare Bauteil über eine nach distal gerade fortgesetzte Mantelfläche des Schafts insbesondere nicht oder nicht wesentlich hinaus.

Im Fall eines kreiszylindrischen Schafts ragt das bewegbare Bauteil also in der ersten Konfiguration insbesondere nicht oder nicht wesentlich über die Mantelfläche desjenigen Kreiszylinders hinaus, dessen Mantelfläche die Mantelfläche des Schafts enthält. Das bewegbare Bauteil ragt in der ersten Konfiguration nicht wesentlich über die nach distal gerade fortgesetzte Mantelfläche des Schafts hinaus, wenn es um nicht mehr als ein Fünftel oder nicht mehr als ein Zehntel des Durchmessers des Schafts hinausragt.

Ein Endoskop, wie es hier beschrieben ist, umfasst insbesondere ferner einen Sensor an dem bewegbaren Bauteil.

Ein Endoskop, wie es hier beschrieben ist, umfasst insbesondere ferner einen Sensor an einer Stirnfläche des bewegbaren Bauteils.

Der Sensor ist insbesondere zur Messung eines Drucks, einer Temperatur, eines pH-Werts, einer anderen Konzentration oder eines anderen chemischen Parameters oder eines physikalischen Messwerts vorgesehen und ausgebildet. Der Sensor kann einen Laser umfassen, beispielsweise zum Abtasten einer inneren Oberfläche eines Hohlraums. Der Sensor ist insbesondere unter der Stirnfläche und angrenzend an die Stirnfläche des bewegbaren Bauteils angeordnet. Der Sensor ist insbesondere dauerhaft und nicht zerstörungsfrei lösbar mit dem bewegbaren Bauteil verbunden oder in das bewegbare Bauteil integriert.

Bei einem Endoskop, wie es hier beschrieben ist, umfasst die erste optische Einrichtung an der Stirnfläche des Schafts insbesondere ein Objektiv oder eine andere Abbildungseinrichtung zum Erzeugen eines reellen Bilds eines Objekts.

Die erste optische Einrichtung kann ferner einen Bildsensor zum Erfassen des reellen Bilds oder ein Relaislinsensystem oder ein geordnetes Bündel von Lichtleitfasern oder eine andere Bildübertragungseinrichtung zum Übertragen des von dem Objektiv oder der anderen Abbildungseinrichtung erzeugten reellen Bilds umfassen.

Bei einem Endoskop, wie es hier beschrieben ist, ist die zweite optische Einrichtung insbesondere an der Stirnfläche des bewegbaren Bauteils angeordnet.

Eine Anordnung der zweiten optischen Einrichtung an der Stirnfläche des bewegbaren Bauteils, die in der ersten Konfiguration der Stirnfläche an dem distalen Ende des Schafts gegenüber angeordnet ist, kann eine Anordnung der zweiten optischen Einrichtung neben der ersten optischen Einrichtung in der zweiten Konfiguration ermöglichen.

Wenn das bewegbare Bauteil ferner einen Sensor umfasst, sind die zweite optische Einrichtung und der Sensor insbesondere an voneinander abgewandten Stirnflächen des bewegbaren Bauteils angeordnet.

Bei einem Endoskop, wie es hier beschrieben ist, weist das bewegbare Bauteil insbesondere im Wesentlichen die Gestalt eines Zylinders mit zwei Stirnflächen und einer Mantelfläche auf, wobei die zweite optische Einrichtung an der Mantelfläche des bewegbaren Bauteils angeordnet ist.

Das bewegbare Bauteil weist insbesondere die Gestalt eines Kreiszylinders auf. Die zweite optische Einrichtung ist insbesondere so an der Mantelfläche des bewegbaren Bauteils angeordnet, dass in der zweiten Konfiguration eine Lichteintrittsfläche oder Lichtaustrittsfläche der zweiten optischen Einrichtung an einer von dem Schaft abgewandten Seite des bewegbaren Bauteils angeordnet ist.

Bei einem Endoskop, wie es hier beschrieben ist, umfasst die zweite optische Einrichtung an dem bewegbaren Bauteil insbesondere ein Objektiv oder eine andere Abbildungseinrichtung zum Erzeugen eines reellen Bilds eines Objekts.

Bei einem Endoskop, wie es hier beschrieben ist, umfasst die zweite optische Einrichtung an dem bewegbaren Bauteil insbesondere ein Objektiv oder eine andere Abbildungseinrichtung zum Erzeugen eines reellen Bilds eines Objekts, wobei die erste optische Einrichtung und die zweite optische Einrichtung zur Erfassung von unterschiedlichen Wellenlängenbereichen ausgebildet sind.

Die zweite optische Einrichtung an dem bewegbaren Bauteil umfasst insbesondere ferner einen Bildsensor zum Erfassen des durch das Objektiv oder die andere Abbildungseinrichtung erzeugten reellen Bilds.

Insbesondere sind die erste optische Einrichtung zum Erfassen von Licht innerhalb des gesamten für das gesunde menschliche Auge sichtbaren Wellenlängenbereichs und die zweite optische Einrichtung weitgehend oder ausschließlich zum Erfassen von Fluoreszenzlicht innerhalb eines engen Wellenlängenbereichs (im infraroten, im sichtbaren oder im ultravioletten Wellenlängenbereich) oder umgekehrt vorgesehen und ausgebildet.

Bei einem Endoskop, wie es hier beschrieben ist, ist eine Winkelposition des bewegbaren Bauteils relativ zu dem distalen Ende des Schafts insbesondere innerhalb eines vorbestimmten Winkelbereichs veränderbar.

Bei einem Endoskop, wie es hier beschrieben ist, umfasst die zweite optische Einrichtung an dem bewegbaren Bauteil insbesondere ein Objektiv oder eine andere Abbildungseinrichtung zum Erzeugen eines reellen Bilds eines Objekts, wobei eine Winkelposition des bewegbaren Bauteils relativ zu dem distalen Ende des Schafts innerhalb eines vorbestimmten Winkelbereichs veränderbar ist, um einen Schnittpunkt zwischen einer optischen Achse der Abbildungseinrichtung der ersten optischen Einrichtung und einer optischen Achse der Abbildungseinrichtung der zweiten optischen Einrichtung zu bewegen oder um einen von der Abbildungseinrichtung der ersten optischen Einrichtung und der Abbildungseinrichtung der zweiten optischen Einrichtung erfassten Raumwinkelbereich zu vergrößern oder zu verkleinern.

Wenn die erste optische Einrichtung an dem distalen Ende des Schafts und die zweite optische Einrichtung an dem bewegbaren Bauteil gemeinsam zum Erfassen eines Stereobilds vorgesehen und ausgebildet sind, kann durch Schwenken des bewegbaren Bauteils relativ zu dem distalen Ende des Schafts und damit Schwenken der optischen Achse der Abbildungseinrichtung der zweiten optischen Einrichtung in dem bewegbaren Bauteil relativ zu der optischen Achse der Abbildungseinrichtung der ersten optischen Einrichtung in dem distalen Ende des Schafts der Schnittpunkt zwischen beiden optischen Achsen bewegt werden. Damit kann die Fläche (oft als Nullebene bezeichnet) verschoben werden, in der Gegenstände liegen, die ohne Disparität erfasst werden.

Wenn die erste optische Einrichtung an dem distalen Ende des Schafts und die zweite optische Einrichtung an dem bewegbaren Bauteil zum Erfassen von überlappenden oder aneinander angrenzenden Bereichen vorgesehen und ausgebildet sind, kann durch Bewegen des bewegbaren Bauteils relativ zu dem distalen Ende des Schafts die Größe des durch beide optische Einrichtungen zusammen erfassten Gesamtbereichs verändert werden.

Ein Endoskop, wie es hier beschrieben ist, umfasst insbesondere ferner eine dritte optische Einrichtung.

Ein Endoskop, wie es hier beschrieben ist, umfasst insbesondere ferner eine dritte optische Einrichtung, wobei die dritte optische Einrichtung ein Objektiv oder eine andere Einrichtung zum Erzeugen eines reellen Bilds eines Objekts umfasst.

Die dritte optische Einrichtung ist insbesondere an dem bewegbaren Bauteil angeordnet. Die dritte optische Einrichtung kann ferner einen Bildsensor zum Erfassen des von dem Objektiv oder der anderen Einrichtung zum Erzeugen eines reellen Bilds erzeugten reellen Bilds umfassen.

Bei einem Endoskop, wie es hier beschrieben ist, weist das bewegbare Bauteil insbesondere eine von der Stirnfläche abgewandte weitere Stirnfläche auf, wobei die dritte optische Einrichtung an der weiteren Stirnfläche des bewegbaren Bauteils angeordnet ist.

Ein Endoskop, wie es hier beschrieben ist, umfasst insbesondere ferner ein weiteres bewegbares Bauteil, das mit dem bewegbaren Bauteil gelenkig verbunden ist, wobei die dritte optische Einrichtung an dem weiteren bewegbaren Bauteil angeordnet ist.

Insbesondere sind das bewegbare Bauteil und das weitere bewegbare Bauteil jeweils zylinderförmig oder im Wesentlichen zylinderförmig mit einer Mantelfläche und zwei voneinander abgewandten Stirnflächen, wobei die zweite optische Einrichtung an der Mantelfläche des bewegbaren Bauteils und die dritte optische Einrichtung an der Mantelfläche des weiteren bewegbaren Bauteils angeordnet sind.

Bei einem Endoskop, wie es hier beschrieben ist, weist insbesondere zumindest entweder die erste optische Einrichtung oder die zweite optische Einrichtung mehrere Lichtquellen zur Erzeugung von Licht unterschiedlicher spektraler Eigenschaften auf.

Insbesondere sind jeweils eine oder mehrere Lichtquellen zum Erzeugen von Licht, das von dem gesunden menschlichen Auge als weiß empfunden wird, und eine oder mehrere Lichtquellen zum Erzeugen von Licht zur Anregung von Fluoreszenz und/oder für therapeutische Zwecke vorgesehen.

Ein Endoskop, wie es hier beschrieben ist, umfasst insbesondere ferner einen Drucksensor oder eine Laser-Messeinrichtung oder eine andere Messeinrichtung zum Erfassen von einem oder mehreren Messwerten an dem bewegbaren Bauteil oder an dem distalen Ende des Schafts.

Bei einem Endoskop, wie es hier beschrieben ist, weist der Schaft insbesondere eine erste Kontaktfläche auf, wobei das weitere bewegbare Bauteil eine zu der ersten Kontaktfläche komplementär geformte zweite Kontaktfläche aufweist, wobei in der ersten Konfiguration die zweite Kontaktfläche von der ersten Kontaktfläche beabstandet ist, und wobei in der zweiten Konfiguration die zweite Kontaktfläche an dem weiteren bewegbaren Bauteil die erste Kontaktfläche an dem Schaft flächig berührt.

Bei einem Endoskop, entsprechend der vorliegenden Erfindung, weist der Schaft eine erste Kontaktfläche auf, wobei das bewegbare Bauteil eine zu der ersten Kontaktfläche komplementär geformte zweite Kontaktfläche aufweist, wobei in der ersten Konfiguration die zweite Kontaktfläche von der ersten Kontaktfläche beabstandet ist, und wobei in der zweiten Konfiguration die zweite Kontaktfläche die erste Kontaktfläche flächig berührt.

Ein Endoskop umfasst einen Schaft zum Einführen in einen zu betrachtenden Hohlraum, ein bewegbares Bauteil, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, eine erste Kontaktfläche an dem Schaft und eine zu der ersten Kontaktfläche komplementär geformte zweite Kontaktfläche an dem bewegbaren Bauteil, wobei in der ersten Konfiguration die zweite Kontaktfläche von der ersten Kontaktfläche beabstandet ist, und wobei in der zweiten Konfiguration die zweite Kontaktfläche die erste Kontaktfläche flächig berührt.

Die erste Kontaktfläche und die zweite Kontaktfläche sind jeweils insbesondere eben oder zylindrisch (beispielsweise kreiszylindrisch) und weisen die gleiche Krümmung auf, wobei eine der beiden Kontaktflächen konvex und die andere konkav ausgebildet ist. Die flächige Berührung der Kontaktflächen ermöglicht eine Wärmeübertragung oder Wärmeleitung von dem bewegbaren Bauteil zu dem Schaft. In dem bewegbaren Bauteil erzeugte Abwärme kann an den Schaft übertragen und in dem Schaft zu dem proximalen Ende des Endoskops und damit aus dem betrachteten Hohlraum herausgeleitet werden.

Ein Endoskop, wie es hier beschrieben ist, umfasst insbesondere ferner eine Heatpipe oder eine andere Wärmeleiteinrichtung in dem Schaft, die mit der ersten Kontaktfläche unmittelbar wärmeleitfähig verbunden ist.

Die Heatpipe oder andere Wärmeleiteinrichtung kann eine Übertragung von Abwärme, die in dem bewegbaren Bauteil freigesetzt wird, zu dem proximalen Ende des Endoskops und damit aus dem betrachteten Hohlraum heraus ermöglichen. Alternativ oder zusätzlich kann die Heatpipe oder andere Wärmeleiteinrichtung eine Verteilung der Abwärme und damit eine Abfuhr der Abwärme bei geringerer Oberflächentemperatur des Schafts ermöglichen. Optional kann die Heatpipe oder andere Wärmeleiteinrichtung ferner Abwärme, die an dem distalen Ende des Schafts entsteht, zu dem proximalen Ende des Endoskops übertragen oder entlang des Schafts verteilen.

Ein Endoskop, wie es hier beschrieben ist, umfasst insbesondere ferner ein elastisches Bauteil, das das bewegbare Bauteil mit dem distalen Ende des Schafts mechanisch verbindet.

Das elastische Bauteil kann eine gelenkige Verbindung zwischen dem bewegbaren Bauteil und dem distalen Ende des Schafts schaffen und dabei insbesondere wie ein Festkörpergelenk wirken. Die Elastizität des elastischen Bauteils rührt von den mechanischen, insbesondere den elastischen Eigenschaften des Materials oder der Materialien des elastischen Bauteils und von seiner Gestalt her. Die Gestalt des elastischen Bauteils kann insbesondere eine Bewegbarkeit des bewegbaren Bauteils relativ zu dem distalen Ende des Schafts überwiegend oder fast ausschließlich um eine vorbestimmte Schwenkachse ermöglichen. Das elastische Bauteil kann beispielsweise Federstahl, Nitinol oder eine andere Nickel-Titan-Legierung oder eine andere pseudoelastische Metalllegierung oder einen Kunststoff aufweisen oder vollständig aus einem dieser Materialien gebildet sein.

Ein Endoskop, wie es hier beschrieben ist, umfasst insbesondere ferner ein wärmeleitfähiges elastisches Bauteil, das sowohl in der ersten Konfiguration als auch in der zweiten Konfiguration das bewegbare Bauteil mit dem distalen Ende des Schafts wärmeleitfähig verbindet.

Ein Endoskop umfasst einen Schaft zum Einführen in einen zu betrachtenden Hohlraum, ein bewegbares Bauteil, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, und ein wärmeleitfähiges elastisches Bauteil, das sowohl in der ersten Konfiguration als auch in der zweiten Konfiguration das bewegbare Bauteil mit dem distalen Ende des Schafts wärmeleitfähig verbindet.

Das wärmeleitfähige elastische Bauteil kann eine Abfuhr von in dem bewegbaren Bauteil freigesetzter Abwärme zu dem distalen Ende des Schafts ermöglichen oder unterstützen. Optional kann das wärmeleitfähige elastische Bauteil zusätzlich eine gelenkige mechanische Verbindung des bewegbaren Bauteils mit dem distalen Ende des Schafts schaffen, ähnlich wie ein Festkörpergelenk.

Das wärmeleitfähige elastische Bauteil ist insbesondere ein Bauteil, wie es als "thermal strap" angeboten und vertrieben wird. Das wärmeleitfähige elastische Bauteil ist insbesondere teilweise oder vollständig aus Kupfer, Silber, Aluminium, Gold, Graphit, Graphen, Diamant, Kohlenstoffnanoröhren, Siliziumcarbid, Aluminiumnitrid oder einem anderen Material, dessen Wärmeleitfähigkeit mindestens die Hälfte der Wärmeleitfähigkeit von reinem Kupfer, d. h. mindestens 200 W/Km beträgt, gebildet.

Das wärmeleitfähige elastische Bauteil verbindet das bewegbare Bauteil mit dem distalen Ende des Schafts in der ersten Konfiguration, in der zweiten Konfiguration und in allen weiteren, insbesondere zwischen der ersten Konfiguration und der zweiten Konfiguration liegenden Konfigurationen. Das wärmeleitfähige elastische Bauteil ist insbesondere wärmeleitfähig verbunden mit einem distalen Ende einer Heatpipe oder einer anderen Wärmeleiteinrichtung, die in dem Schaft angeordnet ist, um Abwärme zu dem proximalen Ende des Endoskops zu übertragen oder entlang des Schafts zu verteilen.

Bei einem Endoskop, wie es hier beschrieben ist, ist insbesondere entweder bei der ersten Konfiguration oder bei der zweiten Konfiguration die in dem elastischen Bauteil gespeicherte elastische Energie minimal.

Wenn keine weitere Kraft auf das bewegbare Bauteil wirkt, bewegt das elastische Bauteil das bewegbare Bauteil in diejenige Konfiguration, in der das elastische Bauteil die geringste elastische Energie enthält. Das elastische Bauteil umfasst beispielsweise ein Federelement in Gestalt einer Helix oder in anderer Gestalt, das aus Federstahl, Nitinol oder einem anderen pseudoelastischen Material besteht.

Das Endoskop kann ein Seil, einen Bowdenzug, einen Draht oder eine andere Kraftübertragungseinrichtung zur Übertragung einer Kraft zu dem bewegbaren Bauteil und zum Bewegen des bewegbaren Bauteils in eine Konfiguration, in der das elastische Bauteil eine höhere elastische Energie aufweist, umfassen.

Ein Endoskop, wie es hier beschrieben ist, umfasst insbesondere ferner einen Außenschaft, der einen zylindrische Hohlraum umschließt, wobei der Außenschaft relativ zu dem Schaft und dem bewegbaren Bauteil bewegbar ist zwischen einer ersten Position, bei der der Schaft und zumindest ein Teil des bewegbaren Bauteils in dem von dem Außenschaft umschlossenen Hohlraum angeordnet sind, und einer zweiten Position, bei der das bewegbare Bauteil außerhalb des von dem Außenschaft umschlossenen Hohlraums angeordnet ist.

Ein Endoskop, wie es hier beschrieben ist, umfasst insbesondere ferner ein elastisches Bauteil, das das bewegbare Bauteil mit dem distalen Ende des Schafts mechanisch verbindet, und einen Außenschaft, der einen Hohlraum umschließt, wobei der Außenschaft relativ zu dem Schaft und dem bewegbaren Bauteil bewegbar ist zwischen einer ersten Position, bei der der Schaft und zumindest ein Teil des bewegbaren Bauteils in dem von dem Außenschaft umschlossenen Hohlraum angeordnet sind, und einer zweiten Position, bei der das bewegbare Bauteil außerhalb des von dem Außenschaft umschlossenen Hohlraums angeordnet ist, wobei die in dem elastischen Bauteil gespeicherte elastische Energie bei der zweiten Konfiguration minimal ist.

Ein Endoskop umfasst einen Schaft zum Einführen in einen zu betrachtenden Hohlraum, ein bewegbares Bauteil, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, ein elastisches Bauteil, das das bewegbare Bauteil mit dem distalen Ende des Schafts mechanisch verbindet, und einen Außenschaft, der einen Hohlraum umschließt, wobei der Außenschaft relativ zu dem Schaft und dem bewegbaren Bauteil bewegbar ist zwischen einer ersten Position, bei der der Schaft und zumindest ein Teil des bewegbaren Bauteils in dem von dem Außenschaft umschlossenen Hohlraum angeordnet sind, und einer zweiten Position, bei der das bewegbare Bauteil außerhalb des von dem Außenschaft umschlossenen Hohlraums angeordnet ist, und wobei die in dem elastischen Bauteil gespeicherte elastische Energie bei der zweiten Konfiguration minimal ist.

Der von dem Außenschaft umschlossene Hohlraum ist von dem Außenschaft mantelförmig umschlossen, so dass der Schaft und das bewegbare Bauteil nach distal und optional auch der Schaft oder das proximale Ende des Endoskops nach proximal aus dem von dem Au-ßenschaft mantelförmig umschlossenen Hohlraum herausragen können. Der von dem Au-ßenschaft umschlossene Hohlraum ist insbesondere zylindrisch oder im Wesentlichen zylindrisch. Der Querschnitt des von dem Außenschaft umschlossenen Hohlraums und der Querschnitt des Schafts des Endoskops sind insbesondere so aufeinander abgestimmt, dass der Schaft in dem von dem Außenschaft umschlossenen Hohlraum spiel- und reibungsarm geführt ist und bewegt werden kann.

Wenn der Außenschaft relativ zu dem Schaft des Endoskops in der ersten, distalen Position angeordnet ist, erzwingt er eine Anordnung des bewegbaren Bauteils relativ zu dem distalen Ende des Schafts in der ersten Konfiguration. Wenn der Außenschaft relativ zu dem Schaft in der zweiten, proximalen Position angeordnet ist, kann das bewegbare Bauteil - insbesondere angetrieben durch eine Rückstellkraft des elastischen Bauteils - zu der zweiten Konfiguration bewegt werden.

Die Bewegung des Außenschafts relativ zu dem Schaft und dem bewegbaren Bauteil zwischen der ersten, distalen Position und der zweiten, proximalen Position erfolgt beispielweise manuell, indem medizinisches Personal oder eine andere Person, die das Endoskop verwendet, mit der Hand eine entsprechende Kraft auf das proximale Ende des Außenschafts ausübt.

Ein Endoskop, wie es hier beschrieben ist, umfasst insbesondere ferner ein Zahnrad, das mit dem bewegbaren Bauteil starr verbunden oder auf andere Weise mechanisch gekoppelt ist, und eine in dem Schaft bewegbare Zahnstange oder Schnecke, die mit dem Zahnrad kämmt, so dass eine Bewegung der Zahnstange oder Schnecke mit einer Bewegung des bewegbaren Bauteils einhergeht.

Ein Endoskop umfasst einen Schaft zum Einführen in einen zu betrachtenden Hohlraum, ein bewegbares Bauteil, das an dem distalen Ende des Schafts angeordnet und relativ zu dem distalen Ende des Schafts bewegbar ist zwischen einer ersten Konfiguration, in der der Schaft in einen Hohlraum einführbar ist, und einer zweiten Konfiguration, die zur Betrachtung des Hohlraums vorgesehen ist, ein Zahnrad, das mit dem bewegbaren Bauteil starr verbunden oder auf andere Weise mechanisch gekoppelt ist, und eine in dem Schaft bewegbare Zahnstange oder Schnecke, die mit dem Zahnrad kämmt, so dass eine Bewegung der Zahnstange oder Schnecke mit einer Bewegung des bewegbaren Bauteils einhergeht.

Entsprechend dem vorgesehenen Bewegungsbereich des bewegbaren Bauteils relativ zu dem distalen Ende des Schafts muss das Zahnrad nicht an seinem gesamten Umfang Zähne aufweisen. Wenn das bewegbare Bauteil beispielsweise nur um höchstens 180 Grad geschwenkt werden kann, kann das Zahnrad als halbes Zahnrad ausgebildet sein und nur entlang eines Kreisbogens, der einen Winkel von 180 Grad oder etwas mehr überdeckt, Zähne aufweisen.

Das Zahnrad kann nach radial außen ragende Zähne oder in axialer Richtung sich erstreckende Zähne aufweisen. Zähne des Zahnrads können beispielsweise durch Stifte mit kreiszylindrischer oder anderer Gestalt gebildet sein, die an oder nahe einem bogenförmigen Rand einer Scheibe oder zwischen zwei insbesondere parallelen Scheiben angeordnet sein können. Die Rotationsachse des Zahnrads ist insbesondere die Schwenkachse des bewegbaren Bauteils.

Die Zahnstange ist insbesondere für eine Bewegung parallel oder im Wesentlichen parallel zu einer Längsachse des Schafts vorgesehen, ausgebildet und geführt. Zähne der Zahnstange können sich beispielsweise in einer Richtung orthogonal zu der Rotationsachse des Zahnrads oder in einer Richtung parallel zu der Rotationsachse des Zahnrads erstrecken. Zähen der Zahnstange können durch Bereiche zwischen Nuten in der Zahnstange oder durch kreiszylindrische oder andere zylindrische Bereiche der Zahnstange gebildet sein.

Die Schnecke ist insbesondere um eine Achse rotierbar, die parallel oder im Wesentlichen parallel zu der Längsachse des Schafts des Endoskops ist.

Ein Endoskop, wie es hier beschrieben ist, umfasst insbesondere ferner ein manuell bewegbares Bedienteil an einem proximalen Ende des Endoskops, eine Kurvenscheibe, die mit dem manuell bewegbaren Bedienteil mechanisch gekoppelt ist, und ein Seil oder einen Bowdenzug oder einen Draht oder eine Zug- oder Druckstange oder eine andere Kraftübertragungseinrichtung, die die Kurvenscheibe mit dem bewegbaren Bauteil koppelt.

Das bewegbare Bedienteil ist insbesondere relativ zu anderen Teilen des Endoskops bewegbar. Das bewegbare Bedienteil ist beispielsweise ein Drehrad oder ein Scherengriff.

Die Kurvenscheibe ist insbesondere unmittelbar mechanisch starr mit dem bewegbaren Bedienteil verbunden. Alternativ kann die Kurvenscheibe über ein Getriebe mit dem bewegbaren Bedienteil gekoppelt sein.

Die Kurvenscheibe weist beispielsweise eine Rille auf, in der ein Seil, ein Bowdenzug oder ein Draht geführt ist, dessen Ende mit der Kurvenscheibe mechanisch starr verbunden sein kann. Alternativ kann die Kurvenscheibe ähnlich wie ein Nocken an einer Nockenwelle auf ein elastisch gegen die Kurvenscheibe gepresstes proximales Ende einer Zug- oder Druckstange wirken. Alternativ kann die Kurvenscheibe eine Nut aufweisen, in die beispielsweise ein Stift an einem proximalen Ende einer Zug- oder Druckstange eingreift.

Ein Endoskop, wie es hier beschrieben ist, umfasst insbesondere ferner einen Scherengriff an einem proximalen Ende des Endoskops mit zwei relativ zueinander bewegbaren Griffteilen, wobei die Griffteile derart mit dem Schaft und mit dem bewegbaren Bauteil gekoppelt sind, dass eine Relativbewegung der Griffteile mit einer Bewegung des bewegbaren Bauteils relativ zu dem Schaft einhergeht.

Insbesondere ist eines der zwei relativ zueinander bewegbaren Griffteile unmittelbar oder mittelbar mit einem proximalen Ende des Schafts des Endoskops mechanisch starr verbunden. In diesem Fall ist das andere der zwei Griffteile insbesondere durch ein Seil, einen Bowdenzug, einen Draht, eine Zug- oder Druckstange oder eine andere Kraftübertragungseinrichtung mit dem bewegbaren Bauteil gekoppelt.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine weitere schematische Darstellung des Endoskops aus Figur 1;
- Figur 3: eine weitere schematische Darstellung des Endoskops aus den Figuren 1 und 2;
- Figur 4: eine weitere schematische Darstellung des Endoskops aus den Figuren 1 bis 3;
- Figur 5: eine weitere schematische Darstellung des Endoskops aus den Figuren 1 bis 4;
- Figur 6: eine schematische Darstellung einer Variante des Endoskops aus den Figuren 1 bis 5;
- Figur 7: eine weitere schematische Darstellung des Endoskops aus Figur 6;
- Figur 8: eine schematische Darstellung einer weiteren Variante des Endoskops aus den Figuren 1 bis 5;
- Figur 9: eine schematische Darstellung einer weiteren Variante des Endoskops aus den Figuren 1 bis 5;
- Figur 10: eine schematische Darstellung einer weiteren Variante des Endoskops aus den Figuren 1 bis 5;
- Figur 11: eine schematische Darstellung einer weiteren Variante des Endoskops aus den Figuren 1 bis 5;
- Figur 12: eine schematische Darstellung einer weiteren Variante des Endoskops aus den Figuren 1 bis 5;
- Figur 13: eine schematische Darstellung einer weiteren Variante des Endoskops aus den Figuren 1 bis 5;
- Figur 14: eine schematische Darstellung einer weiteren Variante des Endoskops aus den Figuren 1 bis 5;
- Figur 15: eine weitere schematische Darstellung des Endoskops aus Figur 14;
- Figur 16: eine schematische Darstellung einer weiteren Variante des Endoskops aus den Figuren 1 bis 5;
- Figur 17: eine schematische Darstellung einer weiteren Variante des Endoskops aus den Figuren 1 bis 5;
- Figur 18: eine schematische Darstellung einer weiteren Variante des Endoskops aus den Figuren 1 bis 5;
- Figur 19: eine weitere schematische Darstellung des Endoskops aus Figur 18.

### Beschreibung der Ausführungsformen

Figur 1 zeigt eine schematische Darstellung eines Endoskops 10 mit einem proximalen Ende 12 und einem distalen Ende 14. Eine Handhabungseinrichtung 16 des Endoskops 10 bildet dessen proximales Ende 12.

Bei dem dargestellten Beispiel umfasst die Handhabungseinrichtung 16 einen Bereich mit vergrößertem Querschnitt, in dem elektronische, optoelektronische, elektrooptische, optische und andere Baugruppen angeordnet sein können. Die Handhabungseinrichtung 16 kann mit ihrem vergrößerten Querschnitt ein manuelles Greifen, Halten und Führen des Endoskops 10 vereinfachen und/oder eine Befestigung an einem Stativ oder an einem manuell oder motorisch verstellbaren Arm (in letzterem Fall oft als Roboterarm bezeichnet) ermöglichen.

An der Handhabungseinrichtung können - wie in Figur 1 rechts angedeutet - ein oder mehrere Steckverbinder zur Verbindung des Endoskops 10 mit einer elektrischen Leistungsquelle, einer Lichtquelle, einer Kamerakontrolleinheit (oft als CCU bezeichnet) oder mit andere Geräten oder Instrumenten durch entsprechende Leitungen vorgesehen sein.

Das Endoskop 10 umfasst ferner einen Schaft 20, dessen proximales Ende 22 mit der Handhabungseinrichtung 16 verbunden ist oder in diese übergeht. Ein distales Ende 23 des Schafts 20 ist nahe dem distalen Ende 14 des Endoskops angeordnet.

Bei dem dargestellten Beispiel ist der Schaft 20 gerade und starr. Die Mantelfläche 26 des Schafts 20 stellt einen Ausschnitt aus der Mantelfläche eines Zylinders, insbesondere eines Kreiszylinders dar. Als Längsachse 28 des Schafts 20 wird die Symmetrieachse dieses Kreiszylinders bezeichnet, auf der die Flächenschwerpunkte aller Querschnitte des Schafts 20 liegen.

Alternativ kann der Schaft teilweise oder vollständig gekrümmt oder flexibel und somit krümmbar ausgebildet sein. Für diese Fälle ist im Folgenden mit der Längsachse 28 des Schafts 20 immer die Längsachse des Schafts 20 nahe dessen distalem Ende 23 gemeint.

Ferner kann der Schaft 20 alternativ nicht-kreisförmige Querschnitte aufweisen. In diesem Fall ist die Längsachse insbesondere die Linie, auf der die Flächenschwerpunkte der Querschnitte des Schafts liegen.

Der Schaft 20 weist an seinem distalen Ende 23 eine Stirnfläche 24 auf, die bei dem dargestellten Beispiel eben und orthogonal zu der Längsachse 28 des Schafts 20 ist. Alternativ und abweichend davon kann die Stirnfläche 24 an dem distalen Ende 23 des Schafts 20 gekrümmt und/oder relativ zu der Längsachse 28 des Schafts 20 geneigt sein.

Das Endoskop 10 weist ferner ein bewegbares Bauteil 30 auf, das durch ein in Figur 1 nicht dargestellte Gelenk mit dem distalen Ende 23 des Schafts 20 gelenkig verbunden und relativ zu dem distalen Ende 23 des Schafts 20 schwenkbar ist. Das Gelenk definiert eine Schwenkachse 38 orthogonal zu der Zeichenebene der Figur 1, um die das bewegbare Bauteil 30 relativ zu dem distalen Ende 23 des Schafts 20 schwenkbar ist. Das Gelenk kann ein formschlüssiges Gelenk mit einer Welle oder einem Achszapfen und einem korrespondierenden Lager oder ein Festkörpergelenk sein. Das Gelenk kann als Koppelgetriebe ausgestaltet sein, bei dem mit einer Schwenkbewegung eine Translationsbewegung einhergeht, ähnlich wie dies von verdeckt eingebauten Scharnieren im Möbelbau bekannt ist.

Bei dem dargestellten Beispiel liegt die Schwenkachse 38 an oder nahe den Rändern der Stirnfläche 24 an dem distalen Ende 23 des Schafts 20 und der ersten Stirnfläche 32 des bewegbaren Bauteils 30. Das bewegbare Bauteil 30 ist relativ zu dem distalen Ende 23 des Schafts 20 innerhalb eines vorbestimmten Winkelbereichs zwischen der in Figur 1 dargestellten ersten Konfiguration und einer in Figur 2 gezeigten zweiten Konfiguration schwenkbar. Bei der in Figur 1 gezeigten ersten Konfiguration des Endoskops 10 ist das bewegbare Bauteil 30 distal des distalen Endes 23 des Schafts 20 angeordnet.

Bei dem dargestellten Beispiel weist das bewegbare Bauteil 30 im Wesentlichen die Gestalt eines kurzen Kreiszylinders mit einer ersten Stirnfläche 32, einer zweiten Stirnfläche 34 und einer Mantelfläche 36 auf. Die Stirnflächen 32, 34 des bewegbaren Bauteils 30 sind bei dem dargestellten Beispiel eben und parallel oder im Wesentlichen parallel zueinander und orthogonal oder im Wesentlichen orthogonal zu der Mantelfläche 36. Der Querschnitt des bewegbaren Bauteils 30 entspricht im Wesentlichen dem Querschnitt des Schafts 20.

In der in Figur 1 dargestellten ersten Konfiguration des Endoskops bildet die Mantelfläche 36 des bewegbaren Bauteils 30 eine gerade und im Wesentlichen glatte und im Wesentlichen spaltfrei sich distal anschließende Verlängerung der Mantelfläche 26 des Schafts 20. In dieser ersten Konfiguration des Endoskops 10 kann der Schaft 20 durch eine Trokarhülse oder durch eine andere Öffnung mit einem Querschnitt, der nicht oder nicht wesentlich größer als der Querschnitt des Schafts 20 und des bewegbaren Bauteils 30 sein muss, in einen zu betrachtenden oder zu untersuchenden Hohlraum eingeführt werden.

Eine erste optische Einrichtung 50 des Endoskops 10 ist an dem distalen Ende 23 des Schafts 20 angeordnet. Die erste optische Einrichtung 50 ist unmittelbar proximal der Stirnfläche 24 an dem distalen Ende 23 des Schafts 20 angeordnet und grenzt an die Stirnfläche 24 an. Eine Lichteintrittsfläche 56 der ersten optischen Einrichtung 50 ist Teil der Stirnfläche 24 am distalen Ende 23 des Schafts 20.

Die erste optische Einrichtung 50 umfasst insbesondere ein Objektiv oder eine andere Abbildungseinrichtung zum Erzeugen eines reellen Bilds. Ferner kann die erste optische Einrichtung 50 einen oder mehrere Bildsensoren zur Erfassung des von dem Objektiv oder der anderen Abbildungseinrichtung der ersten optischen Einrichtung 50 erzeugten Bilds aufweisen. Alternativ kann ein Relaislinsensystem oder ein geordnetes Bündel von Lichtleitfasern oder eine andere Bildübertragungseinrichtung in dem Schaft 20 angeordnet sein, um das von dem Objektiv oder der anderen Abbildungseinrichtung der ersten optischen Einrichtung 50 erzeugte reelle Bild zu dem proximalen Ende 12 des Endoskops 10 zu übertragen.

Alternativ oder zusätzlich kann die erste optische Einrichtung 50 eine oder mehrere Leuchtdioden oder andere Lichtquellen zum Erzeugen von Beleuchtungslicht und/oder eine Lichtaustrittsfläche eines Lichtleiters zum Übertragen von Beleuchtungslicht umfassen. Eine oder mehrere Lichtaustrittsflächen der Lichtquelle oder der Lichtquellen oder des Lichtleiters oder der Lichtleiter können ebenfalls Teil der Stirnfläche 24 am distalen Ende 23 des Schafts 20 sein.

Eine zweite optische Einrichtung 60 ist in dem bewegbaren Bauteil 30 und unmittelbar angrenzend an deren erste Stirnfläche 32 angeordnet. Eine oder mehrere Lichteintrittsflächen 66 der zweiten optischen Einrichtung 60 sind Teil der ersten Stirnfläche 32 des bewegbaren Bauteils 30.

Die zweite optische Einrichtung 60 umfasst insbesondere ein Objektiv oder eine andere Abbildungseinrichtung zum Erzeugen eines reellen Bilds und einen oder mehrere Bildsensoren zum Erfassen des von dem Objektiv oder der anderen Abbildungseinrichtung erzeugten reellen Bilds.

Alternativ oder zusätzlich kann die zweite optische Einrichtung 60 eine oder mehrere Leuchtdioden oder andere Lichtquellen zum Erzeugen von Beleuchtungslicht und/oder eine oder mehrere Lichtaustrittsflächen von Lichtleitern zum Übertragen von Beleuchtungslicht umfassen. Eine oder mehrere Lichtaustrittsflächen der Lichtquelle oder der Lichtquellen oder des Lichtleiters oder der Lichtleiter können ebenfalls Teil der ersten Stirnfläche 32 des bewegbaren Bauteils 30 sein.

Eine dritte optische Einrichtung 70 ist in dem bewegbaren Bauteil 30 unmittelbar angrenzend an die zweite Stirnfläche 34 des bewegbaren Bauteils 30 angeordnet. Eine oder mehrere Lichteintrittsflächen 76 der dritten optischen Einrichtung 70 sind Teil der zweiten Stirnfläche 34 des bewegbaren Bauteils 30.

Die dritte optische Einrichtung 70 umfasst insbesondere ein Objektiv oder eine andere Abbildungseinrichtung zum Erzeugen eines reellen Bilds und einen oder mehrere Bildsensoren zum Erfassen des von dem Objektiv oder der anderen Abbildungseinrichtung erzeugten reellen Bilds.

Alternativ oder zusätzlich kann die dritte optische Einrichtung 70 eine oder mehrere Leuchtdioden oder andere Lichtquellen zum Erzeugen von Beleuchtungslicht und/oder eine oder mehrere Lichtaustrittsflächen von Lichtleitern zum Übertragen von Beleuchtungslicht umfassen. Eine oder mehrere Lichtaustrittsflächen der Lichtquelle oder der Lichtquellen oder des Lichtleiters oder der Lichtleiter können ebenfalls Teil der zweiten Stirnfläche 34 des bewegbaren Bauteils 30 sein.

Bei dem dargestellten Beispiel sind die Stirnfläche 24 an dem distalen Ende 23 des Schafts 20 und die Stirnflächen 32, 34 des bewegbaren Bauteils 30 jeweils eben oder im Wesentlichen eben. Die Stirnfläche 24 an dem distalen Ende 23 des Schafts 20 ist orthogonal oder im Wesentlichen orthogonal zu der Längsachse 28 des Schafts 20. Die Stirnflächen 32, 34 des bewegbaren Bauteils 30 sind parallel zueinander und bei der in Figur 1 dargestellten Konfiguration parallel zu der Stirnfläche 24 an dem distalen Ende 23 des Schafts 20 und orthogonal zu der Längsachse 28 des Schafts 20.

Bei der in Figur 1 dargestellten Konfiguration liegt die erste Stirnfläche 32 des bewegbaren Bauteils 30 unmittelbar gegenüber der Stirnfläche 24 an dem distalen Ende 23 des Schafts 20 oder an dieser teilweise oder vollständig an. Die erste optische Einrichtung 50 an dem distalen Ende 23 des Schafts 20 und die zweite optische Einrichtung 60 in dem bewegbaren Bauteil 30 haben deshalb in der in Figur 1 dargestellten Konfiguration keine Funktion und sind insbesondere deaktiviert.

Die dritte optische Einrichtung 70 weist eine optische Achse 78 auf, die insbesondere durch das Objektiv oder die andere Abbildungseinrichtung der dritten optischen Einrichtung 70 definiert ist. Die optische Achse 78 stellt insbesondere gleichzeitig die Blickrichtung der dritten optischen Einrichtung 70 dar. Durch gestrichelte Linien ist ferner das Sichtfeld der dritten optischen Einrichtung 70 angedeutet. Objekte, die innerhalb dieses Sichtfelds der dritten optischen Einrichtung 70 liegen, werden durch die dritte optische Einrichtung insbesondere beleuchtet und erfasst. Die dritte optische Einrichtung 70 ermöglicht so insbesondere in der in Figur 1 dargestellten Konfiguration des Endoskops 10 ein Einführen des distalen Endes 14 des Endoskops 10 in einen Hohlraum unter optischer Kontrolle.

Figur 2 zeigt eine weitere schematische Darstellung des Endoskops 10 aus Figur 1. Die Art der Darstellung, insbesondere die Orientierung der Zeichenebene, entspricht derjenigen der Figur 1.

In Figur 2 ist eine zweite Konfiguration des Endoskops 10 dargestellt, die sich von der in Figur 1 dargestellten ersten Konfiguration dadurch unterscheidet, dass das bewegbare Bauteil 30 um einen Winkel von ca. 180 Grad um die Schwenkachse 38 geschwenkt ist. Das bewegbare Bauteil 30 ist deshalb nicht mehr distal des distalen Endes 23 des Schafts 20, sondern neben dem distalen Ende 23 des Schafts 20 angeordnet. Die Mantelfläche 36 des bewegbaren Bauteils 30 liegt linienförmig an der Mantelfläche 26 des Schafts 20 an oder ist von dieser entlang einer geraden Linie nur geringfügig beabstandet.

Bei der in Figur 2 gezeigten zweiten Konfiguration sind die Stirnfläche 24 an dem distalen Ende 23 des Schafts 20 mit der Lichteintrittsfläche 56 der ersten optischen Einrichtung 50 einerseits und die erste Stirnfläche 32 des bewegbaren Bauteils 30 mit der Lichteintrittsfläche 66 der zweiten optischen Einrichtung 60 andererseits nebeneinander in einer Ebene angeordnet. Bei dem dargestellten Beispiel sind die optische Achse 58 der ersten optischen Einrichtung 50 und die optische Achse 68 der zweiten optischen Einrichtung 60 parallel oder im Wesentlichen parallel zueinander. Wenn sowohl die erste optische Einrichtung 50 als auch die zweite optische Einrichtung 60 jeweils zum Erfassen eines Bilds vorgesehen und ausgebildet sind, kann in der in Figur 2 gezeigten zweiten Konfiguration des Endoskops 10 ein Stereobild erfasst werden.

Bei der in Figur 2 gezeigten zweiten Konfiguration des Endoskops 10 ist die Blickrichtung der dritten optischen Einrichtung 70 nach proximal orientiert. Dies kann eine Orientierung innerhalb eines Hohlraums vereinfachen. Insbesondere kann mittels der dritten optischen Einrichtung 70 die Umgebung einer Öffnung, durch die der Schaft 20 des Endoskops 10 in einen Hohlraum hineinragt, erfasst werden. Alternativ kann die dritte optische Einrichtung 70 in der in Figur 2 gezeigten Konfiguration deaktiviert oder abgeschaltet werden.

Figur 3 zeigt eine weitere schematische Darstellung des anhand der Figuren 1 und 2 dargestellten Endoskops 10 in einer weiteren Konfiguration, die der anhand der Figur 2 dargestellten Konfiguration ähnelt. Die Art der Darstellung, insbesondere die Zeichenebene der Figur 3 entspricht derjenigen der Figuren 1 und 2.

Die in Figur 3 gezeigte Konfiguration unterscheidet sich von der anhand der Figur 2 dargestellten Konfiguration insbesondere dadurch, dass das bewegbare Bauteil 30 ausgehend von der anhand der Figur 1 dargestellten ersten Konfiguration um einen Winkel, der kleiner als 180 Grad ist, um die Schwenkachse 38 geschwenkt ist. Die optischen Achsen 68, 78 der zweiten optischen Einrichtung 60 und der dritten optischen Einrichtung 70 in dem bewegbaren Bauteil 30 sind deshalb nicht parallel zu der Längsachse 28 des Schafts 20 und zu der optischen Achse 58 der ersten optischen Einrichtung 50 an dem distalen Ende 23 des Schafts 20. Stattdessen schneiden die optische Achse 58 der ersten optischen Einrichtung am dem distalen Ende 23 des Schafts 20 einerseits und die optische Achse 68 der zweiten optischen Einrichtung 60 in dem bewegbaren Bauteil 30 andererseits einander in einem Schnittpunkt. In einem Stereobild weisen die Bilder von Objekten in dem Schnittpunkt der optischen Achsen 58, 68 und in einer Fläche (oft idealisiert als Nullebene bezeichnet), die diesen Schnittpunkt enthält, keine Disparität auf.

Ferner liegt bei der in Figur 3 gezeigten Konfiguration die Mantelfläche 36 des bewegbaren Bauteils 30 nicht an der Mantelfläche 26 des Schafts 20 an. Stattdessen liegt ein keilförmiger Spalt zwischen dem bewegbaren Bauteil 30 und dem Schaft 20 vor.

Alternativ und abweichend von der anhand der Figuren 1 bis 3 dargestellten zylindrischen Gestalt des Schafts 20 und des bewegbaren Bauteils 30 können der Schaft 20 an seinem distalen Ende 23 und/oder das Bauteil 30 keilförmig ausgebildet sein, so dass bei der in Figur 3 gezeigten relativen Orientierung der optischen Achsen 58, 68 das bewegbare Bauteil 30 trotzdem an dem Schaft 20 linienförmig oder flächig anliegt.

Alternativ können die optische Achse 58 der ersten optischen Einrichtung 50 an dem distalen Ende 23 des Schafts 20 gegenüber der Längsachse 28 des Schafts 20 und/oder die optische Achse 68 der zweiten optischen Einrichtung 60 in dem bewegbaren Bauteil 30 gegenüber der Symmetrieachse der Mantelfläche 36 des bewegbaren Bauteils 30 geneigt sein. In diesem Fall können auch bei der anhand der Figur 2 gezeigten zweiten Konfiguration (bewegbares Bauteil 30 parallel zu dem Schaft 20) die optischen Achsen 58, 68 wie in Figur 3 gezeigt einander schneiden. Dazu können insbesondere auch die Lichteintrittsfläche 56 der ersten optischen Einrichtung 50 an dem distalen Ende 23 des Schafts 20 gegenüber der Längsachse 28 des Schafts 20 und/oder die Lichteintrittsfläche 66 der zweiten optischen Einrichtung 60 gegenüber der Symmetrieachse der Mantelfläche 36 des bewegbaren Bauteils 30 gekippt sein.

Figur 4 zeigt eine weitere schematische Darstellung des anhand der Figuren 1 bis 3 dargestellten Endoskops 10 in einer weiteren Konfiguration. Die Art der Darstellung, insbesondere die Zeichenebene der Figur 4 entspricht derjenigen der Figuren 1 bis 3.

Die in Figur 4 gezeigte Konfiguration unterscheidet sich von den anhand der Figuren 2 und 3 dargestellten Konfigurationen dadurch, dass das bewegbare Bauteil 30 relativ zu dem Schaft 20 eine andere Winkelposition einnimmt. Der Winkel zwischen der optischen Achse 68 der zweiten optischen Einrichtung 60 in dem bewegbaren Bauteil 30 und der optischen Achse 58 der ersten optischen Einrichtung 50 an dem distalen Ende 23 des Schafts 20 ist größer als bei der anhand der Figur 3 dargestellten Konfiguration. Deshalb ist der Abstand des Schnittpunkts der optischen Achsen 58, 68 von dem distalen Ende 14 des Endoskops 10 bei der in Figur 4 gezeigten Konfiguration kleiner als bei der anhand der Figur 3 dargestellten Konfiguration.

Durch eine Variation des Winkels zwischen dem bewegbaren Bauteil 30 und dem Schaft 20 kann die Position des Schnittpunkts der optischen Achsen 58, 68 und damit der ohne Disparität im Stereobild erfassten Fläche ("Nullebene") verändert werden.

Figur 5 zeigt eine weitere schematische Darstellung des anhand der Figuren 1 bis 4 dargestellten Endoskops in einer weiteren Konfiguration. Die Art der Darstellung, insbesondere die Zeichenebene der Figur 5 entspricht derjenigen der Figuren 1 bis 4.

Die in Figur 5 gezeigte Konfiguration unterscheidet sich von den anhand der Figuren 1 bis 4 dargestellten Konfigurationen dadurch, dass die Winkelposition des bewegbaren Bauteils 30 relativ zu dem Schaft 20 bei der in Figur 5 gezeigten Konfiguration zwischen den Winkelpositionen bei den anhand der Figuren 1 und 4 dargestellten Konfigurationen liegt. Der Winkel zwischen dem bewegbaren Bauteil 30 und dem Schaft 20 ist somit noch größer als bei der anhand der Figur 4 dargestellten Konfiguration. Entsprechend ist auch der Winkel zwischen der optischen Achse 58 der ersten optischen Einrichtung 50 an dem distalen Ende 23 des Schafts 20 und der optischen Achse 68 der zweiten optischen Einrichtung 60 in dem bewegbaren Bauteil 30 noch größer als bei der anhand der Figur 4 dargestellten Konfiguration. Der Abstand des Schnittpunkts der optischen Achsen 58, 68 von dem distalen Ende 14 des Endoskops 10 ist sehr klein im Verhältnis zu der Länge der Basis, d. h. dem Abstand der Mittelpunkt der Lichteintrittsflächen 56, 66 der ersten optischen Einrichtung 50 und der zweiten optischen Einrichtung 60. Deshalb wäre eine Betrachtung eines mittels der optischen Einrichtungen 50, 60 in der in Figur 5 gezeigten Konfiguration erfassten Stereobilds unangenehm bis schmerzhaft oder würde nicht mehr zu einem dreidimensionalen Eindruck führen.

Die in Figur 5 gezeigte Konfiguration kann jedoch eine gleichzeitige Erfassung eines vergrößerten Raumwinkelbereichs ermöglichen. Dazu kann insbesondere ein von der ersten optischen Einrichtung 50 an dem distalen Ende 23 des Schafts 20 erzeugtes Bild und ein von der zweiten optischen Einrichtung 60 in dem bewegbaren Bauteil 30 erzeugtes Bild aneinandergefügt werden.

Bei der in Figur 5 gezeigten Konfiguration des Endoskops 10 sind die Blickrichtung 68 der zweiten optischen Einrichtung 60 und die Blickrichtung 78 der dritten optischen Einrichtung nicht ganz orthogonal zu der Längsachse 28 des Schafts 20. Die Blickrichtung der zweiten optischen Einrichtung 60 ist etwas mehr nach distal orientiert, die Blickrichtung der dritten optischen Einrichtung 70 ist etwas mehr nach proximal orientiert. Deshalb überlappen die ringförmigen Bereiche, die bei einer Rotation des Endoskops 10 um die Längsachse 28 des Schafts 20 einerseits mittels der zweiten optischen Einrichtung 60 und andererseits mittels der dritten optischen Einrichtung 70 in dem bewegbaren Bauteil 30 erfassbar sind, nur wenig. Umgekehrt bedeutet dies, dass durch Rotation des Endoskops 10 um die Längsachse 28 des Schafts 20 bei der in Figur 5 gezeigten Konfiguration ein großer zusammenhängender Raumbereich erfasst werden kann.

Figur 6 zeigt eine schematische Darstellung einer Variante des anhand der Figuren 1 bis 5 dargestellten Endoskops. Die Art der Darstellung, insbesondere die Zeichenebene der Figur 6 entspricht derjenigen der Figuren 1 bis 5. Das in Figur 6 gezeigte Endoskop ähnelt in einigen Merkmalen, Eigenschaften und Funktionen dem anhand der Figuren 1 bis 5 dargestellten Endoskop. Nachfolgend sind insbesondere Merkmale, Eigenschaften und Funktionen des in Figur 6 gezeigten Endoskops 10 beschrieben, in denen dieses sich von dem anhand der Figuren 1 bis 5 dargestellten Endoskop unterscheidet.

Das in Figur 6 gezeigte Endoskop 10 unterscheidet sich von dem anhand der Figuren 1 bis 5 dargestellten Endoskop insbesondere dadurch, dass es nicht nur ein bewegbares Bauteil, sondern zwei bewegbare Bauteile 30, 40 aufweist. Das erste bewegbare Bauteil 30 ist gelenkig und um eine erste Schwenkachse 38 relativ zu dem distalen Ende 23 des Schafts 20 schwenkbar mit dem distalen Ende 23 des Schafts 20 mechanisch verbunden. Das zweite bewegbare Bauteil 40 ist gelenkig und um eine zweite Schwenkachse 48 relativ zu dem ersten bewegbaren Bauteil 30 schwenkbar mit dem ersten bewegbaren Bauteil 30 verbunden.

Bei der in Figur 6 gezeigten ersten Konfiguration sind das erste bewegbare Bauteil 30 - ähnlich wie bei der anhand der Figur 1 dargestellten Konfiguration - distal des distalen Endes 23 des Schafts 20 und das zweite bewegbare Bauteil 40 distal des ersten bewegbaren Bauteils 30 angeordnet.

Bei der in Figur 6 gezeigten Konfiguration ragen das erste bewegbare Bauteil 30 und das zweite bewegbare Bauteil 40 nicht oder nicht wesentlich über die nach distal gerade fortgesetzte Mantelfläche 26 des Schafts 20 hinaus. Insbesondere sind die Querschnitte des Schafts 20, des ersten bewegbaren Bauteils 30 und des zweiten bewegbaren Bauteils 40 gleich oder im Wesentlichen gleich. Die Mantelfläche 26 des Schafts 20, die Mantelfläche 36 des ersten bewegbaren Bauteils 30 und die Mantelfläche 46 des zweiten bewegbaren Bauteils 40 gehen - von kleinen Spalten zwischen dem distalen Ende des Schafts 23 und dem ersten bewegbaren Bauteil 30 und zwischen dem ersten bewegbaren Bauteil 30 und dem zweiten bewegbaren Bauteil 40 abgesehen - glatt ineinander über.

In der in Figur 6 gezeigten ersten Konfiguration kann der Schaft 20 mit den bewegbaren Bauteilen 30, 40 voraus durch eine Öffnung, deren Querschnitt nicht oder nicht wesentlich größer als die einzelnen Querschnitte des Schafts 20 und der bewegbaren Bauteile 30, 40 sein muss, in einen Hohlraum eingeführt werden.

Ausgehend von der in Figur 6 gezeigten ersten Konfiguration können das zweite bewegbare Bauteil 40 relativ zu dem ersten bewegbaren Bauteil 30 um die zweite Schwenkachse 48 und/oder das erste bewegbare Bauteil 30 relativ zu dem distalen Ende 23 des Schafts 20 um die erste Schwenkachse 38 geschwenkt werden. Die Schwenkbewegung des zweiten bewegbaren Bauteils 40 relativ zu dem ersten bewegbaren Bauteil 30 um die zweite Schwenkachse 48 kann unabhängig von der Schwenkbewegung des ersten bewegbaren Bauteils 30 relativ zu dem distalen Ende 23 des Schafts 20 um die erste Schwenkachse 38 sein. Alternativ kann eine Kopplung derart vorgesehen sein, dass eine Schwenkbewegung des ersten bewegbaren Bauteils 30 relativ zu dem distalen Ende 23 des Schafts 20 um die erste Schwenkachse 38 mit einer Schwenkbewegung des zweiten bewegbaren Bauteils 40 relativ zu dem ersten bewegbaren Bauteil 30 um die zweite Schwenkachse 48 einhergeht.

An dem Schaft 20 ist eine Kontaktfläche 27 vorgesehen. An der Mantelfläche 46 des zweiten bewegbaren Bauteils 40 ist eine zu der Kontaktfläche 27 an dem Schaft 20 korrespondierende Kontaktfläche 47 vorgesehen.

Das in Figur 6 gezeigte Endoskop 10 unterscheidet sich von dem anhand der Figuren 1 bis 5 dargestellten Endoskop ferner dadurch, dass eine zweite optische Einrichtung 60 und eine dritte optische Einrichtung 70 nicht an Stirnflächen des ersten bewegbaren Bauteils 30 oder des zweiten bewegbaren Bauteils 40 angeordnet sind. Vielmehr ist die zweite optische Einrichtung 60 an der Mantelfläche 36 des ersten bewegbaren Bauteils 30 angeordnet, und die dritte optische Einrichtung 70 ist an der Mantelfläche 46 des zweiten bewegbaren Bauteils 40 angeordnet. Bei der in Figur 6 gezeigten Konfiguration des Endoskops 10 sind Blickrichtungen der zweiten optischen Einrichtung 60 und der dritten optischen Einrichtung 70 orthogonal oder im Wesentlichen orthogonal zu der Längsachse 28 des Schafts 20.

Figur 7 zeigt eine weitere schematische Darstellung des Endoskops 10 aus Figur 6. Die Art der Darstellung, insbesondere die Zeichenebene der Figur 7 entspricht derjenigen der Figuren 1 bis 6. Das Endoskop 10 ist in Figur 7 in einer zweiten Konfiguration gezeigt, die sich von der anhand der Figur 6 dargestellten ersten Konfiguration unterscheidet.

Bei der in Figur 7 gezeigten zweiten Konfiguration des Endoskops 10 sind das zweite bewegbare Bauteil 40 relativ zu dem ersten bewegbaren Bauteil 30 um einen vorbestimmten Winkel (beispielsweise 60 Grad) um die zweite Schwenkachse 48 und das erste bewegbare Bauteil 30 relativ zu dem distalen Ende 23 des Schafts 20 um einen vorbestimmten Winkel (beispielsweise 150 Grad) um die erste Schwenkachse 38 geschwenkt. Bei der so erreichten, in Figur 7 gezeigten zweiten Konfiguration liegt die Kontaktfläche 47 an dem zweiten bewegbaren Bauteil 40 an der korrespondierenden Kontaktfläche 27 an dem Schaft 20 an. Dies kann eine Übertragung von Abwärme, die beispielsweise in der dritten optischen Einrichtung 70 in dem zweiten bewegbaren Bauteil 40 freigesetzt wird, zu dem Schaft 20 ermöglichen oder vereinfachen.

Bei dem anhand der Figuren 6 und 7 dargestellten Beispiel sind die optischen Einrichtungen 50, 60, 70 und deren Sichtfelder so ausgebildet und die bewegbaren Bauteile 30, 40 bei der in Figur 7 gezeigten zweiten Konfiguration so angeordnet und orientiert, dass die Sichtfelder der optischen Einrichtungen 50, 60, 70 im Arbeitsabstand (außerhalb des in Figur 7 dargestellten Bereichs) einander überlappen. Deshalb kann in der in Figur 7 gezeigten zweiten Konfiguration mit den optischen Einrichtungen 50, 60, 70 zusammen gleichzeitig eine Weitwinkelaufnahme der Umgebung des distalen Endes 14 des Endoskops 10 erfasst werden. Mit einer Rotation des Endoskops 10 um die Längsachse 28 des Schafts 20 kann die innere Oberfläche eines Hohlraums nahezu vollständig erfasst werden. Bei den in Figur 7 angedeuteten Sichtfeldern kann lediglich ein kleiner Bereich rund um den Schaft 20 des Endoskops 10 in den Hohlraum ragt, nicht erfasst werden.

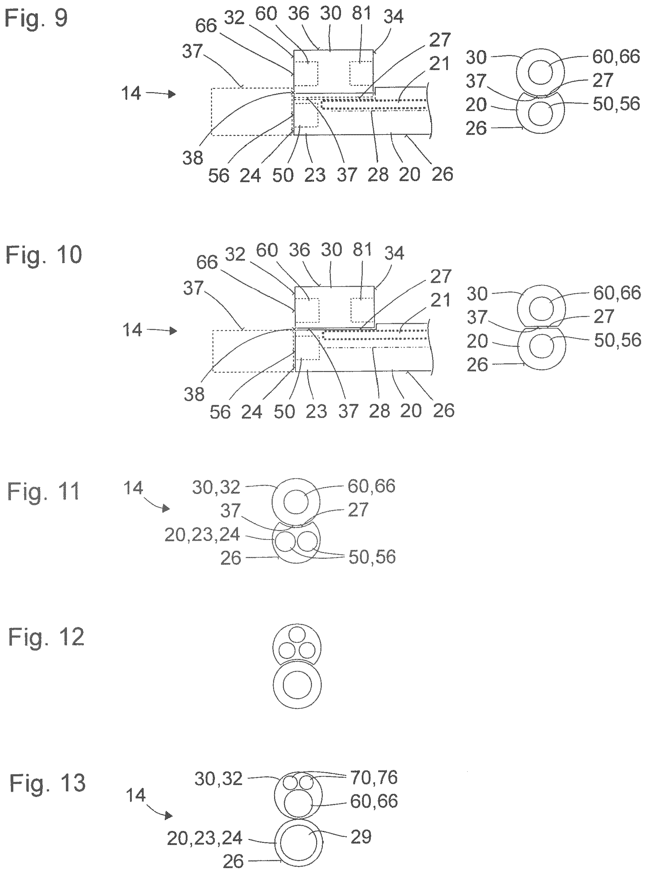

Figur 8 zeigt eine schematische Darstellung eines Schnitts durch ein distales Ende 14 einer weiteren Variante des anhand der Figuren 1 bis 5 dargestellten Endoskops. Die Schnittebene der Figur 8 ist parallel zu den Zeichenebenen der Figuren 1 bis 7. Das distale Ende 14 des Endoskops ist in Figur 9 vergrößert gegenüber den Darstellungen in den Figuren 1 bis 7 gezeigt. In Figur 8 ist die anhand der Figur 1 dargestellte Konfiguration gezeigt.

Das in Figur 8 dargestellte Endoskop ähnelt in einigen Merkmalen, Eigenschaften und Funktionen dem anhand der Figuren 1 bis 5 dargestellten Endoskop. Nachfolgend sind insbesondere Merkmale, Eigenschaften und Funktionen des in Figur 8 gezeigten Endoskops beschrieben, in denen dieses sich von dem anhand der Figuren 1 bis 5 dargestellten Endoskop unterscheidet.

In Figur 8 ist angedeutet, dass jede der optischen Einrichtungen 50, 60, 70 ein Objektiv 52, 62, 72 und einen Bildsensor 54, 64, 74 umfasst. Alternativ kann jede optische Einrichtung 50, 60, 70 mehrere Bildsensoren 52, 62, 72 für unterschiedliche Wellenlängenbereiche und dichroitisch reflektierende Flächen ausweisen, so dass auf jeden Bildsensor 54, 64, 74 nur Licht innerhalb eines vorbestimmten Wellenlängenbereichs fällt. Jede optische Einrichtung 50, 60, 70 kann ferner mehrere Objektive 52, 62, 72 und mehrere Bildsensoren 54, 64, 74 aufweisen, um ein Stereobild zu erfassen oder Bilder in mehreren verschiedenen Wellenlängenbereichen zu erfassen. Jede der optischen Einrichtungen 50, 60, 70 kann alternativ oder zusätzlich eine oder mehrere Leuchtdioden oder andere Lichtquellen zum Erzeugen von Beleuchtungslicht und/oder eine oder mehrere Lichtaustrittsflächen von Lichtleitern zum Übertragen von Beleuchtungslicht aufweisen.

Auch bei den anhand der Figuren 1 bis 7 dargestellten Endoskopen können die optischen Einrichtungen an dem distalen Ende des Schafts und in dem bewegbaren Bauteil jeweils ähnlich aufgebaut sein wie hier beispielhaft anhand der Figur 8 beschrieben.

Wie bereits insbesondere für das anhand der Figuren 6 und 7 beschriebene Endoskop erwähnt, kann in einem bewegbaren Bauteil freigesetzte Abwärme über einen flächigen mechanischen Kontakt zu dem Schaft übertragen werden. Zusätzlich kann in dem Schaft jeweils eine Heatpipe oder eine andere Einrichtung zum Übertragen von Wärme vorgesehen sein, um Abwärme entlang des Schafts zu dem proximalen Ende des Endoskops zu übertragen.

Bei dem in Figur 8 dargestellten Beispiel ist ein wärmeleitfähiges elastisches Bauteil 94 vorgesehen, das das bewegbare Bauteil 30 mit dem distalen Ende 23 des Schafts 20 wärmeleitfähig verbindet. Das wärmeleitfähige elastische Bauteil 94 ist insbesondere unmittelbar oder über ein anderes wärmeleitfähiges Bauteil mittelbar mit einer oder mehreren Wärmequellen des bewegbaren Bauteils 30 verbunden, beispielsweise - wie in Figur 8 angedeutet - mit der zweiten optischen Einrichtung 60, optional auch mit der dritten optischen Einrichtung 70. Das andere Ende des wärmeleitfähigen elastischen Bauteils 94 ist beispielsweise mit einem distalen Ende einer Heatpipe 21 oder einer anderen Wärmeübertragungseinrichtung in dem Schaft 20 verbunden.

Das wärmeleitfähige elastische Bauteil 94 umfasst insbesondere ein Gewebe oder Geflecht oder Bündel aus dünnen und elastischen wärmeleitfähigen Fasern. Das wärmeleitfähige elastische Bauteil 94 weist beispielsweise Kupfer, Silber, Aluminium, Gold, Graphit, Graphen, Diamant, Kohlenstoffnanoröhren, Siliziumcarbid, Aluminiumnitrid oder ein anderes Material, dessen Wärmeleitfähigkeit mindestens die Hälfte der Wärmeleitfähigkeit von reinem Kupfer - d. h. mindestens 200 W/Km - beträgt, auf. Das wärmeleitfähige elastische Bauteil 94 ist insbesondere ein wärmeleitfähiges elastisches Bauteil, wie es kommerziell unter dem Begriff "Thermal Strap" angeboten und vertrieben wird.

Das wärmeleitfähige elastische Bauteil 94 kann gegenüber einer Wärmeübertragung mittels aneinander anliegenden Kontaktflächen, wie sie anhand der Figuren 6 und 7 dargestellt sind, den Vorteil aufweisen, in vielen verschiedenen Winkelpositionen des bewegbaren Bauteils 30 relativ zu distalen Ende 23 des Schafts 20 eine Abfuhr von Abwärme aus dem bewegbaren Bauteil 30 zu ermöglichen.

Die Heatpipe 21 in dem Schaft 20 kann eine Übertragung der Abwärme aus dem bewegbaren Bauteil 30 und optional auch von Abwärme der ersten optischen Einrichtung 50 oder einer anderen Wärmequelle an dem distalen Ende 23 des Schafts 20 zu dem proximalen Ende 12 des Endoskops 10 (vgl. Figuren 1 bis 7) ermöglichen. So können das wärmeleitfähige elastische Bauteil 94 und die Heatpipe 21 die Oberflächentemperatur des Endoskops an seinem distalen Ende 14 und die in den mittels des Endoskops zu untersuchenden Hohlraum eingebrachte Wärmeleistung senken.

Das wärmeleitfähige elastische Bauteil 94 kann gleichzeitig - ähnlich einem Festkörpergelenk - die einzige mechanische Verbindung des bewegbaren Bauteils 30 mit dem distalen Ende 23 des Schafts 20 bilden. Alternativ kann zusätzlich zu dem wärmeleitfähigen elastischen Bauteil 94 ein anderes Gelenk vorgesehen sein.

Figur 9 zeigt zwei schematische Darstellungen des distalen Endes 14 einer weiteren Variante des anhand der Figuren 1 bis 5 dargestellten Endoskops. Das distale Ende 14 des Endoskops ist in Figur 9 vergrößert gegenüber den Darstellungen in den Figuren 1 bis 7 gezeigt, ähnlich wie in Figur 8. Davon abgesehen ähnelt die Art der Darstellung, insbesondere die Zeichenebene, in Figur 9 links derjenigen der Figuren 1 bis 7. In Figur 9 rechts ist das distale Ende 14 des Endoskops in einer Ansicht von distal gezeigt. Die Zeichenebene der Figur 9 rechts ist somit orthogonal zu der Zeichenebene der Figur 9 links und zu der Längsachse 28 des Schafts 20.

Das Endoskop, dessen distales Ende 14 in Figur 9 dargestellt ist, ähnelt in einigen Merkmalen, Eigenschaften und Funktionen den anhand der Figuren 1 bis 5 und 8 dargestellten Endoskopen. Nachfolgend sind insbesondere Merkmale, Eigenschaften und Funktionen des Endoskops, dessen distales Ende 14 in Figur 9 gezeigt ist, beschrieben.

In Figur 9 ist das bewegbare Bauteil 30 in durchgezogenen Linien in seiner Position, die der anhand der Figur 2 dargestellten zweiten Konfiguration des Endoskops entspricht, dargestellt. In Figur 9 links ist das bewegbare Bauteil 30 ferner in seiner Position, die der anhand der Figur 1 dargestellten ersten Konfiguration des Endoskops entspricht, in gestrichelten Linien angedeutet.

Das Endoskop, dessen distales Ende 14 in Figur 9 gezeigt ist, unterscheidet sich von den anhand der Figuren 1 bis 8 dargestellten Endoskopen insbesondere dadurch, dass der Schaft 20 nahe seinem distalen Ende 23 eine erste, konkave Kontaktfläche 27 in einer flachen Ausnehmung aufweist. Die Gestalt der ersten Kontaktfläche 27 an dem distalen Ende 23 des Schafts 20 korrespondiert zu der Gestalt eines als zweite Kontaktfläche 37 an dem bewegbaren Bauteil 30 vorgesehenen Bereichs der Mantelfläche 36 des bewegbaren Bauteils 30. Die erste Kontaktfläche 27 an dem distalen Ende 23 des Schafts 20 und die zweite Kontaktfläche 37 an dem bewegbaren Bauteil 30 weisen korrespondierende Krümmungen auf, wobei die erste Kontaktfläche 27 an dem distalen Ende 23 des Schafts 20 konkav und die zweite Kontaktfläche 37 an dem bewegbaren Bauteil 30 konvex ist.

Bei der in Figur 9 in durchgezogenen Linien dargestellten zweiten Konfiguration des Endoskops liegt die zweite Kontaktfläche 37 an dem bewegbaren Bauteil 30 flächig an der ersten Kontaktfläche 27 an dem distalen Ende 23 des Schafts 20 an. Dies ermöglicht einen guten Wärmeübergang von dem bewegbaren Bauteil 30 zu dem distalen Ende 23 des Schafts 20. Bei dem dargestellten Beispiel weisen beide Kontaktflächen 27, 37 jeweils die Gestalt eines Ausschnitts aus der Mantelfläche desselben Kreiszylinders auf.

Das Endoskop, dessen distales Ende 14 in Figur 9 dargestellt ist, unterscheidet sich von dem anhand der Figuren 1 bis 5 dargestellten Endoskop ferner dadurch, dass innerhalb des Schafts 20 (und deshalb nur in gestrichelten Linien angedeutet) eine Heatpipe 21 vorgesehen ist, ähnlich wie bei dem anhand der Figur 8 dargestellten Endoskop. Das distale Ende der Heatpipe 21 ist insbesondere unmittelbar oder mittelbar durch ein wärmeleitfähiges Bauteil mit der ersten Kontaktfläche 27 an dem distalen Ende 23 des Schafts 20 wärmeleitfähig verbunden.

Die Heatpipe 21 ermöglicht eine Ableitung von Abwärme, die in dem bewegbaren Bauteil 30 freigesetzt wird, und optional auch die Ableitung von Abwärme der ersten optischen Einrichtung 50 oder eines anderen Bauteils an dem distalen Ende 23 des Schafts 20 zu dem proximalen Ende des Endoskops.

Das Endoskop, dessen distales Ende 14 in Figur 9 gezeigt ist, unterscheidet sich von den anhand der Figuren 1 bis 8 dargestellten Endoskopen ferner dadurch, dass in dem bewegbaren Bauteil 30 nur eine zweite optische Einrichtung 60 vorgesehen ist. Anstelle der bei den anhand der Figuren 1 bis 6 und 8 dargestellten Endoskopen vorgesehenen dritten optischen Einrichtung ist ein Sensor 81 in dem bewegbaren Bauteil 30 vorgesehen, beispielhaft an der zweiten Stirnfläche 34 des bewegbaren Bauteils 30. Der Sensor 81 ist beispielsweise ein Sensor zur Erfassung eines Drucks, einer Temperatur, eines pH-Werts oder einer anderen Konzentration oder ein Lasersensor zur Abtastung oder anderweitigen Erfassung der Umgebung des distalen Endes 14 des Endoskops.

Figur 10 zeigt eine schematische Darstellung eines distalen Endes 14 einer weiteren Variante des anhand der Figuren 1 bis 5 dargestellten Endoskops. Die Art der Darstellung in Figur 10 entspricht derjenigen in Figur 9. Das Endoskop, dessen distales Ende 14 in Figur 10 dargestellt ist, ähnelt in einigen Merkmalen, Eigenschaften und Funktionen den anhand der Figuren 1 bis 9 dargestellten Endoskopen, insbesondere dem anhand der Figur 9 dargestellten Endoskop. Nachfolgend sind insbesondere Merkmale, Eigenschaften und Funktionen des Endoskops, dessen distales Ende 14 in Figur 10 gezeigt ist, beschrieben, in denen dieses sich von dem anhand der Figur 9 dargestellten Endoskop unterscheidet.

Das Endoskop, dessen distales Ende 14 in Figur 10 gezeigt ist, unterscheidet sich von dem anhand der Figur 9 dargestellten Endoskop insbesondere dadurch, dass beide Kontaktflächen 27, 37 an dem distalen Ende 23 des Schafts 20 und an dem bewegbaren Bauteil 30 jeweils eben ausgebildet sind. Auch dies ermöglicht einen großflächigen Kontakt und einen entsprechend guten Wärmeübergang von dem bewegbaren Bauteil 30 zu dem distalen Ende 23 des Schafts 20.

Figur 11 zeigt eine schematische Darstellung eines distalen Endes 14 einer weiteren Variante des anhand der Figuren 1 bis 5 dargestellten Endoskops. Die Art der Darstellung entspricht derjenigen in den Figuren 9 und 10 jeweils rechts. Die Zeichenebene der Figur 11 ist also orthogonal zu den Zeichenebenen der Figuren 1 bis 8 und zu den Zeichenebenen der Figuren 9 und 10 links und orthogonal zu der Längsachse des Schafts 20. Das distale Ende 14 des Endoskops ist in Figur 11 in der anhand der Figuren 2 dargestellten zweiten Konfiguration des Endoskops gezeigt.

Das Endoskop, dessen distales Ende 14 in Figur 11 gezeigt ist, ähnelt in einigen Merkmalen, Eigenschaften und Funktionen den anhand der Figuren 1 bis 10 dargestellten Endoskopen, insbesondere dem anhand der Figur 9 dargestellten Endoskop. Nachfolgend sind insbesondere Merkmale, Eigenschaften und Funktionen des Endoskops, dessen distales Ende 14 in Figur 11 gezeigt ist, in denen dieses sich von dem anhand der Figur 9 dargestellten Endoskop unterscheidet, beschrieben.

Das in Figur 11 gezeigte Endoskop unterscheidet sich von dem anhand der Figur 9 dargestellten Endoskop insbesondere dadurch, dass an der Stirnfläche 24 an dem distalen Ende 23 des Schafts 20 nebeneinander zwei Lichteintrittsflächen 56 zweier erster optischer Einrichtungen 50 vorgesehen sind. Die beiden ersten optischen Einrichtungen 50 an dem distalen Ende 23 des Schafts 20 sind insbesondere baugleich, weisen also von einer unvermeidbaren Parameterstreuung abgesehen identische Eigenschaften auf, und sind zur Erfassung eines Stereobilds vorgesehen. Alternativ können die beiden ersten optischen Einrichtungen 50 an dem distalen Ende 23 des Schafts 20 zur Erfassung von Bildern bei unterschiedlichen Wellenlängen oder innerhalb unterschiedlicher Wellenlängenbereiche vorgesehen und ausgebildet sein.

Figur 12 zeigt eine schematische Darstellung eines distalen Endes 14 einer weiteren Variante des anhand der Figuren 1 bis 5 dargestellten Endoskops. Die Art der Darstellung entspricht derjenigen in den Figuren 9 und 10 jeweils rechts und der Figur 11. Das distale Ende 14 des Endoskops ist in Figur 12 in der anhand der Figuren 2 dargestellten zweiten Konfiguration des Endoskops gezeigt.

Das Endoskop, dessen distales Ende 14 in Figur 12 gezeigt ist, ähnelt in einigen Merkmalen, Eigenschaften und Funktionen den anhand der Figuren 1 bis 11 dargestellten Endoskopen, insbesondere den anhand der Figuren 9 bis 11 dargestellten Endoskopen. Nachfolgend sind insbesondere Merkmale, Eigenschaften und Funktionen des Endoskops, dessen distales Ende 14 in Figur 12 gezeigt ist, beschrieben, in denen dieses sich von den anhand der Figuren 1 bis 11 dargestellten Endoskopen unterscheidet.

Das Endoskop, dessen distales Ende 14 in Figur 12 gezeigt ist, unterscheidet sich beispielsweise von den anhand der Figuren 9 und 11 dargestellten Endoskopen insbesondere dadurch, dass an der ersten Stirnfläche 32 des bewegbaren Bauteils 30 drei zweite optische Einrichtungen 60 vorgesehen sind. Die drei zweiten optischen Einrichtungen 60 an der ersten Stirnfläche 32 des bewegbaren Bauteils 30 können baugleich sein, beispielsweise zur Erfassung von drei Bildern in dem gleichen Wellenlängenbereich. In diesem Fall können jeweils zwei durch zweite optische Einrichtungen 60 erfasste Bilder ausgewählt werden, um ein Stereobild zu bilden. Alternativ können die zweiten optischen Einrichtungen 60 an der ersten Stirnfläche 32 des bewegbaren Bauteils 30 zur Erfassung von Bildern in unterschiedlichen Wellenlängenbereichen vorgesehen und ausgebildet sein. Alternativ können die zweiten optischen Einrichtungen 60 an der ersten Stirnfläche 32 des bewegbaren Bauteils 30 zur Erzeugung von Beleuchtungslicht gleicher oder unterschiedlicher Spektren vorgesehen sein. Alternativ können ein Teil der zweiten optischen Einrichtungen 60 an der ersten Stirnfläche 32 des bewegbaren Bauteils 30 zur Erfassung von Bildern und ein anderer Teil zum Erzeugen von Beleuchtungslicht vorgesehen und ausgebildet sein.

Das Endoskop, dessen distales Ende 14 in Figur 12 gezeigt ist, unterscheidet sich von den anhand der Figuren 9 und 11 dargestellten Endoskopen ferner dadurch, dass die erste Kontaktfläche 27 an dem distalen Ende 23 des Schafts 20 konvex und die zweite Kontaktfläche 37 an dem bewegbaren Bauteil 30 konkav ausgebildet sind.

Figur 13 zeigt eine schematische Darstellung eines distalen Endes 14 einer weiteren Variante des anhand der Figuren 1 bis 5 dargestellten Endoskops. Die Art der Darstellung entspricht derjenigen in den Figuren 9 und 10 jeweils rechts und der Figuren 11 und 12. Das distale Ende 14 des Endoskops ist in Figur 13 in der anhand der Figuren 2 dargestellten zweiten Konfiguration des Endoskops gezeigt.

Das Endoskop, dessen distales Ende 14 in Figur 13 gezeigt ist, ähnelt in einigen Merkmalen, Eigenschaften und Funktionen den anhand der Figuren 1 bis 11 dargestellten Endoskopen. Nachfolgend sind insbesondere Merkmale, Eigenschaften und Funktionen des Endoskops, dessen distales Ende 14 in Figur 13 gezeigt ist, beschrieben, in denen dieses sich von den anhand der Figuren 1 bis 12 dargestellten Endoskopen unterscheidet.

Das Endoskop, dessen distales Ende 14 in Figur 13 gezeigt ist, unterscheidet sich von den anhand der Figuren 1 bis 12 dargestellten Endoskopen insbesondere dadurch, dass an der ersten Stirnfläche 32 des bewegbaren Bauteils 30 nebeneinander eine zweite optische Einrichtung 60 und zwei dritte optische Einrichtungen 70 vorgesehen sind. Beispielsweise ist die zweite optische Einrichtung 60 zum Erfassen eines Bilds vorgesehen und ausgebildet, und die dritten optischen Einrichtungen 70 sind zum Erzeugen von Beleuchtungslicht vorgesehen und ausgebildet. Dabei können die dritten optischen Einrichtungen 70 baugleich oder unterschiedlich ausgebildet sein, um Beleuchtungslicht unterschiedlichen Spektren bereitzustellen.

Das Endoskop, dessen distales Ende 14 in Figur 13 dargestellt ist, unterscheidet sich von den anhand der Figuren 1 bis 12 dargestellten Endoskopen ferner dadurch, dass an dem distalen Ende 23 des Schafts 20 keine optische Einrichtung vorgesehen ist. Stattdessen weist der Schaft 20 einen Arbeitskanal 29 auf, der in einer Öffnung in der Stirnfläche 24 an dem distalen Ende 23 des Schafts 20 endet. Durch den Arbeitskanal 29 können Fluide oder Festkörper gefördert und/oder ein medizinisches Instrument in einen Hohlraum, in dem das distale Ende 14 des Endoskops angeordnet ist, eingeführt werden.

Auch die anhand der Figuren 1 bis 7 dargestellten Endoskope können Merkmale der anhand der Figuren 8 bis 13 dargestellten Endoskope aufweisen. Beispielsweise können die anhand der Figuren 1 bis 7 dargestellten Endoskope jeweils eine Heatpipe 21 oder eine andere Wärmeleiteinrichtung in oder an dem Schaft 20 aufweisen. Ferner können die anhand der Figuren 1 bis 7 dargestellten Endoskope elastische Bauteile zur gelenkigen mechanischen und/oder wärmeleitfähigen Verbindung des bewegbaren Bauteils oder ersten bewegbaren Bauteils 30 mit dem distalen Ende 23 des Schafts 20 und/oder der bewegbaren Bauteile 30, 40 untereinander aufweisen.

Ferner können die anhand der Figuren 1 bis 10 dargestellten Endoskope Merkmale der anhand der Figuren 11 bis 13 dargestellten Endoskope aufweisen. Insbesondere können an der Stirnfläche 24 am distalen Ende 23 des Schafts 20, an den Stirnflächen 32, 34 des bewegbaren Bauteils 30 und an den Mantelflächen 36, 46 der bewegbaren Bauteile 30, 40 jeweils anstelle einer optischen Einrichtung 50, 60, 70 mehrere baugleiche oder unterschiedliche optische Einrichtungen zum Erzeugen von Beleuchtungslicht und zum Erzeugen und/oder Erfassen reeller Bilder oder ein oder mehrere Sensoren 81 vorgesehen sein.

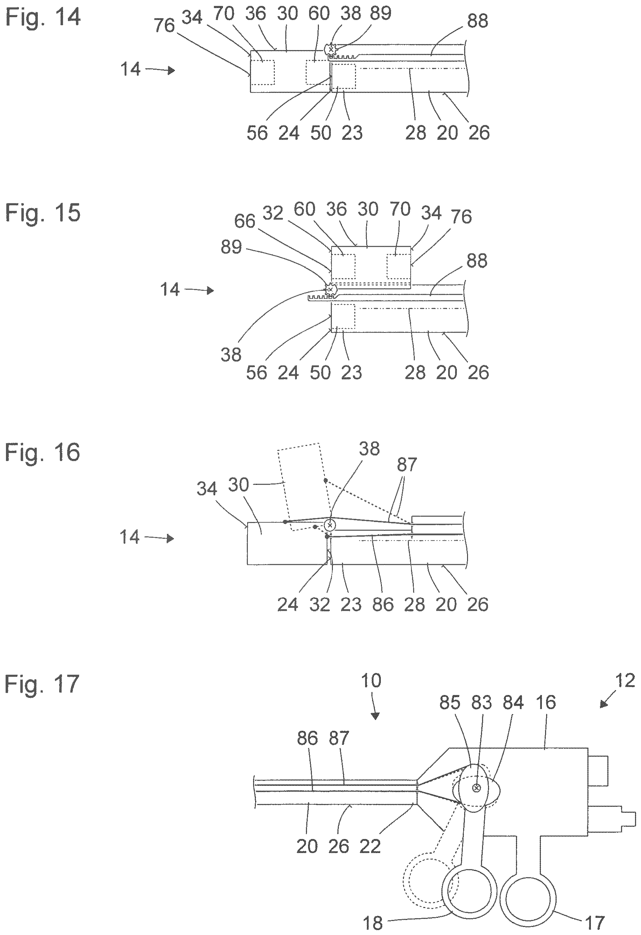

Figur 14 zeigt eine schematische Darstellung eines distalen Endes 14 einer weiteren Variante des anhand der Figuren 1 bis 5 dargestellten Endoskops. Die Art der Darstellung, insbesondere die Zeichenebene entspricht teilweise derjenigen der Figuren 1 bis 7. Das Endoskop, dessen distales Ende 14 in Figur 14 dargestellt ist, ähnelt in einigen Merkmalen, Eigenschaften und Funktionen den anhand der Figuren 1 bis 13 dargestellten Endoskopen. Nachfolgend sind insbesondere Merkmale, Eigenschaften und Funktionen beschriebenen, in denen das Endoskop, dessen distales Ende 14 in Figur 14 dargestellt ist, sich von den anhand der Figuren 1 bis 13 dargestellten Endoskopen unterscheidet.

Das Endoskop, dessen distales Ende 14 in Figur 14 gezeigt ist, weist eine Zahnstange 88 und ein Zahnrad 89 auf. Obwohl die Zahnstange 88 insbesondere innerhalb des Schafts 20 und das Zahnrad 89 insbesondere zumindest teilweise innerhalb des Schafts 20 und des bewegbaren Bauteils 30 angeordnet und deshalb von außen nicht oder nur teilweise sichtbar sind, sind diese in Figur 14 in durchgezogenen Linien vollständig dargestellt, um ihre Funktion erkennbar zu machen.

Die Zahnstange 88 erstreckt sich insbesondere von dem proximalen Ende des Endoskops, wo sie unmittelbar oder mittelbar mit einer Bedieneinrichtung oder einem Antrieb gekoppelt sein kann, bis zu dem distalen Ende 23 des Schafts 20. Das Zahnrad 89 ist unmittelbar oder mittelbar starr mit dem bewegbaren Bauteil 30 verbunden und zusammen mit dem bewegbaren Bauteil 30 um die vorbestimmte Schwenkachse 38 des bewegbaren Bauteils 30 schwenkbar. Alternativ kann das Zahnrad 89 über ein Getriebe oder auf andere Weise mit dem bewegbaren Bauteil 30 gekoppelt sein.

Die Zahnstange 88 weist - insbesondere nur - nahe ihrem distalen Ende im Bereich des Zahnrads 89 Zähne auf, die mit Zähnen des Zahnrads 89 kämmen. Eine Translationsbewegung der Zahnstange 88 (insbesondere parallel zu der Längsachse 28 des Schafts 20) geht deshalb mit einer Schwenkbewegung des bewegbaren Bauteils 30 einher. Bei dem in Figur 14 gezeigten Beispiel liegt die anhand der Figur 1 dargestellte und auch in Figur 14 gezeigte erste Konfiguration des Endoskops vor, wenn die Zahnstange 88 eine äußerst proximale Position einnimmt.

Figur 15 zeigt eine weitere schematische Darstellung des distalen Endes 14 des anhand der Figur 14 dargestellten Endoskops. In Figur 15 ist die anhand der Figur 2 dargestellte zweite Konfiguration des Endoskops gezeigt. Diese zweite Konfiguration wird erreicht, wenn ausgehend von der in Figur 14 gezeigten ersten Konfiguration die Zahnstange 88 in eine äußerst distale, in Figur 15 gezeigte Position bewegt wird.

Im Fall einer starren mechanischen Verbindung des Zahnrads 89 mit dem bewegbaren Bauteil 30 rotiert das Zahnrad 89 beim Übergang zwischen der in Figur 14 dargestellten ersten Konfiguration und der in Figur 15 dargestellten zweiten Konfiguration nur um 180 Grad. Deshalb muss das Zahnrad 89 - wie in den Figuren 14, 15 angedeutet, nicht entlang seines gesamten Umfangs Zähne aufweisen.

Figur 16 zeigt eine schematische Darstellung eines distalen Endes 14 einer weiteren Variante des anhand der Figuren 1 bis 5 dargestellten Endoskops. Die Art der Darstellung in Figur 16, insbesondere die Zeichenebenen, entspricht insbesondere derjenigen der Figuren 14 und 15. Einige Strukturen, die teilweise oder vollständig innerhalb des Schafts 20 und/oder des bewegbaren Bauteils 30 angeordnet und deshalb von außen nicht sichtbar sind, sind in Figur 16 trotzdem in durchgezogenen Linien dargestellt. Das Endoskop, dessen distales Ende 14 in Figur 16 gezeigt ist, ähnelt in einigen Merkmalen, Eigenschaften und Funktionen den anhand der Figuren 1 bis 15 dargestellten Endoskopen. Nachfolgend sind insbesondere Merkmale, Eigenschafen und Funktionen des Endoskops, dessen distales Ende 14 in Figur 16 gezeigt ist, beschrieben, in denen dieses sich von den anhand der Figuren 14 und 15 dargestellten Endoskopen unterscheidet.

Das Endoskop, dessen distales Ende 14 in Figur 16 gezeigt ist, unterscheidet sich beispielsweise von dem anhand der Figuren 14 und 15 dargestellten Endoskop insbesondere dadurch, dass keine Zahnstang und kein Zahnrad zum Bewegen des bewegbaren Bauteils 30 vorgesehen sind. Stattdessen weist das Endoskop ein erstes Zugseil 86 und ein zweites Zugseil 87 auf, deren distale Enden an unterschiedlichen Orten mit dem bewegbaren Bauteil 30 verbunden sind.

Das bewegbare Bauteil 30 und die Zugseile 86, 87 sind in Figur 16 in zwei verschiedenen Positionen dargestellt. In durchgezogenen Linien sind das bewegbare Bauteil 30 und die Zugseile 86, 87 in Positionen gezeigt, die der anhand der Figur 1 dargestellten ersten Konfiguration des Endoskops entsprechen. In gestrichelten Linien sind das bewegbare Bauteil und die Zugseile 86, 87 in Positionen zwischen Positionen der anhand der Figur 1 dargestellten ersten Konfiguration und der anhand der Figuren 2 bis 5 dargestellten zweiten und weiteren Konfigurationen gezeigt.

Eine Zugkraft in dem ersten Zugseil 86 schwenkt das bewegbare Bauteil 30 um die Schwenkachse 38 zu der Position, die der anhand der Figur 1 dargestellten ersten Konfiguration entspricht und in Figur 16 in durchgezogenen Linien dargestellt ist. Eine Zugkraft in dem zweiten Zugseil 87 schwenkt das bewegbare Bauteil 30 über die in Figur 16 in gestrichelten Linien dargestellte Position bis zu der Position, die der anhand der Figur 2 dargestellten zweiten Konfiguration entspricht.

Figur 17 zeigt eine schematische Darstellung des proximalen Endes 12 des Endoskops 10, dessen distales Ende 14 anhand der Figur 16 dargestellt ist. Auch in Figur 17 sind einige Strukturen, die teilweise oder vollständig innerhalb des Endoskops 10 angeordnet und deshalb von außen nicht oder nur teilweise sichtbar sind, in durchgezogenen Linien dargestellt. Im Übrigen entspricht die Darstellung der Figur 17, insbesondere die Zeichenebene, der Darstellung in den Figuren 1 bis 7.

Das Endoskop 10 weist an seinem proximalen Ende 12 ein erstes Griffteil 17 und ein zweites Griffteil 18, die relativ zueinander bewegbar sind, auf. Bei dem dargestellten Beispiel ist das erste Griffteil 17 mechanisch starr mit der übrigen Handhabungseinrichtung 16 und damit mittelbar auch mit dem proximalen Ende 22 des Schafts 20 verbunden. Das zweite Griffteil 18 ist über eine Welle 83, die eine Schwenkachse orthogonal zu der Zeichenebene der Figur 17 definiert, schwenkbar mit der Handhabungseinrichtung 16 verbunden. In der Handhabungseinrichtung 16 sind eine ersten Kurvenscheibe 84 und eine zweite Kurvenscheibe 85 angeordnet, die über die Welle 83 starr (oder alternativ und abweichend von der Darstellung in Figur 17: über ein Getriebe) mit dem zweiten Griffteil 18 gekoppelt sind.

Ein proximales Ende des ersten Zugseils 86 ist mit der ersten Seilscheibe 84 verbunden, ein proximales Ende des zweiten Zugseils 87 ist mit der zweiten Seilscheibe 85 verbunden. Wie in Figur 17 angedeutet, können die Seilscheiben 84, 85 jeweils eine nahezu beliebige und insbesondere von einer Kreisform abweichende Gestalt aufweisen.

Das zweite Griffteil 18, die Seilscheiben 84, 85 und die Zugseile 86, 87 sind jeweils in durchgezogenen Linien in einer ersten Position und in gestrichelten Linien in einer zweiten Position gezeigt. Die Zugseile 86, 87 koppeln das zweite Griffteil 18 derart mit dem bewegbaren Bauteil 30 an dem distalen Ende 23 des Schafts 20 (vgl. Figur 16), dass eine Schwenkbewegung des zweiten Griffteils 18 relativ zu dem ersten Griffteil 17 mit einer Schwenkbewegung des bewegbaren Bauteils 30 um die Schwenkachse 38 einhergeht. Beispielsweise entspricht die in Figur 17 in durchgezogenen Linien dargestellte Position des zweiten Griffteils 18 der in Figur 16 in durchgezogenen Linien dargestellten Position des bewegbaren Bauteils 30, und die in Figur 17 in gestrichelten Linien gezeigte Position des zweiten Griffteils 18 entspricht der in Figur 16 in gestrichelten Linien gezeigten Position des bewegbaren Bauteils 30.

Anstelle des bewegbaren Griffteils 18 kann beispielsweise ein Drehrad, ein Schieber oder ein motorischer Antrieb vorgesehen sein.

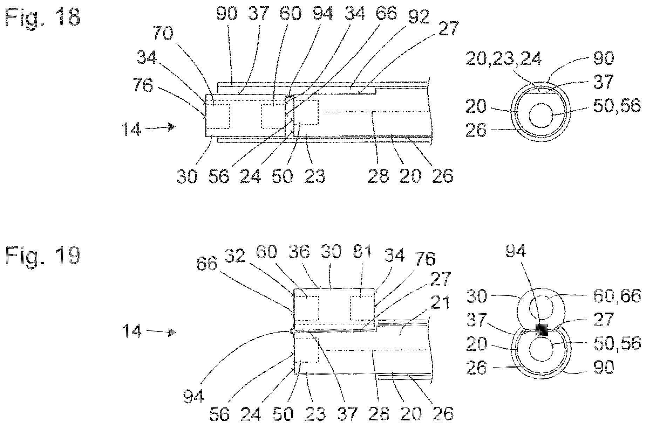

Figur 18 zeigt schematische Darstellungen eines distalen Endes 14 einer weiteren Variante des anhand der Figuren 1 bis 5 dargestellten Endoskops. Die Art der Darstellung, insbesondere die Zeichenebenen entsprechen weitgehend derjenigen der Figuren 9 und 10. Insbesondere sind die Zeichenebene der Figur 18 links parallel zu einer Längsachse 28 des Schafts 20 und die Zeichenebene in Figur 18 rechts orthogonal zu der Zeichenebene in Figur 18 links und orthogonal zu der Längsachse 28 des Schafts 20. Figur 18 links zeigt eine Ansicht von der Seite, wobei abweichend davon ein Außenschaft 90 im Schnitt entlang einer Ebene, die die Längsachse 28 des Schafts 20 enthält und parallel zu der Zeichenebene ist, gezeigt ist. In Figur 18 rechts ist das distale Ende 14 des Endoskops in einer Ansicht von distal gezeigt.

Das Endoskop, dessen distales Ende 14 in Figur 18 gezeigt ist, ähnelt in einigen Merkmalen, Eigenschaften und Funktionen den anhand der Figuren 1 bis 13 dargestellten Endoskopen. Nachfolgend sind insbesondere Merkmale, Eigenschaften und Funktionen des Endoskops, dessen distales Ende 14 in Figur 18 gezeigt ist, in denen dieses sich von den anhand der Figuren 14 bis 17 dargestellten Endoskopen unterscheidet, beschrieben.

Das Endoskop, dessen distales Ende 14 in Figur 18 gezeigt ist, weist ein elastisches Bauteil 94 auf, das das bewegbare Bauteil 30 mit dem distalen Ende 23 des Schafts 20 mechanisch verbindet. Das elastische Bauteil 94 ist so ausgebildet, dass das bewegbare Bauteil 30 relativ zu dem distalen Ende 23 des Schafts 20 ausschließlich oder fast ausschließlich um eine Schwenkachse orthogonal zu der Zeichenebene der Figur 18 links schwenken kann. Dazu ist das elastische Bauteil 94 insbesondere als breites, dünnes und kurzes Band ausgebildet, wobei die Breite in Richtung orthogonal zu der Zeichenebene der Figur 18 links, die Länge in Richtung orthogonal zu der Zeichenebene der Figur 18 rechts und die Dicke in der dritten dazu orthogonalen Richtung gemessen ist.

Das Endoskop, dessen distales Ende 14 in Figur 18 gezeigt ist, weist ferner einen Außenschaft 90 auf, der einen Hohlraum 92 mantelförmig umschließt. In dem von dem Außenschaft 90 mantelförmig umschlossenen Hohlraum 92 sind bei der in Figur 18 dargestellten ersten Konfiguration der Schaft 20 und zumindest ein Teil des bewegbaren Bauteils 30 angeordnet. Der Querschnitt des von dem Außenschaft 90 mantelförmig umschlossenen Hohlraums 92 und die Querschnitte des Schafts 20 und des bewegbaren Bauteils 30 sind so aufeinander abgestimmt, dass der Schaft 20 und das bewegbare Bauteil 30 in dem Außenschaft 90 spiel- und reibungsarm geführt sind.

Bei der in Figur 18 gezeigten ersten Konfiguration des Endoskops ist das bewegbare Bauteil 30 distal des distalen Endes 23 des Schafts 20 angeordnet. Wie erwähnt sind der Schaft 20 und zumindest ein Teil des bewegbaren Bauteils 30 innerhalb des von dem Außenschaft 90 umschlossenen Hohlraums 92 angeordnet. Bei der in Figur 18 gezeigten Position des Außenschafts 90 hält dieser das bewegbare Bauteil 30 in der der ersten Konfiguration entsprechenden Position.

Figur 19 zeigt eine weitere schematische Darstellung des distalen Endes 14 des anhand der Figur 18 dargestellten Endoskops. Die Art der Darstellung, insbesondere die Zeichenebenen, entsprechen derjenigen der Figur 18.

In Figur 19 ist die anhand der Figur 2 dargestellte zweite Konfiguration des Endoskops gezeigt. Der Außenschaft 90 nimmt relativ zu dem Schaft 20 und dem bewegbaren Bauteil 30 eine proximale Position ein, bei der das bewegbare Bauteil 30 und ein distaler Bereich des Schafts 20 außerhalb des von dem Außenschaft 90 mantelförmig umschlossenen Hohlraums 92 und das bewegbare Bauteil 30 neben dem distalen Ende 23 des Schafts 20 angeordnet sind. Das bewegbare Bauteil 30 nimmt die in Figur 19 gezeigte Position ein, weil eine elastische Rückstellkraft des elastischen Bauteils 94 das bewegbare Bauteil 30 in die in Figur 19 gezeigte Position schwenkt. Anders ausgedrückt ist die elastische Energie des elastischen Bauteils 94 in der in Figur 19 gezeigten zweiten Konfiguration geringer als in der in Figur 18 gezeigten ersten Konfiguration.

Eine in den Figuren 18 und 19 nicht gezeigte Ausgestaltung des distalen Rands des Außenschafts 90 kann bei einer von der in Figur 19 gezeigten zweiten Konfiguration ausgehenden Bewegung des Außenschafts 90 nach distal eine Schwenkbewegung des bewegbaren Bauteils 30 gegen die elastische Rückstellkraft des bewegbaren Bauteils 94 bis zu der in Figur 18 gezeigten ersten Konfiguration ermöglichen.

### Bezugszeichen

- **10**: **Endoskop**
- 12: proximales Ende des Endoskops 10
- 14: distales Ende des Endoskops 10
- 16: Handhabungseinrichtung an dem proximalen Ende 12 des Endoskops 10
- 17: erstes Griffteil der Handhabungseinrichtung 16, insbesondere starr mit dem Schaft 20 des Endoskops 10 verbunden
- 18: zweites Griffteil der Handhabungseinrichtung 16, insbesondere mit dem schwenkbaren Bauteil 30 an dem distalen Ende 14 des Endoskops 10 gekoppelt
- **20**: **Schaft des Endoskops 10**
- 21: Heatpipe in dem Schaft 20
- 22: proximales Ende des Schafts 20, mit der Handhabungseinrichtung 16 verbunden
- 23: distales Ende des Schafts 20
- 24: Stirnfläche an dem distalen Ende 23 des Schafts 20
- 26: Mantelfläche des Schafts 20
- 27: Kontaktfläche an dem Schaft 20, zu der Kontaktfläche 37 an dem ersten bewegbaren Bauteil 30 oder zu der Kontaktfläche 47 an dem zweiten bewegbaren Bauteil 40 korrespondierend
- 28: Längsachse des Schafts 20
- 29: Arbeitskanal in dem Schaft 20
- **30**: **(erstes) bewegbares Bauteil**, mit dem distalen Ende 23 des Schafts 20 gelenkig mechanisch verbunden
- 32: erste Stirnfläche des (ersten) bewegbaren Bauteils 30
- 34: zweite Stirnfläche des (ersten) bewegbaren Bauteils 30
- 36: Mantelfläche des (ersten) bewegbaren Bauteils 30
- 37: Kontaktfläche an dem (ersten) bewegbaren Bauteil 30, zu der Kontaktfläche 27 an dem Schaft 20 korrespondierend
- 38: Schwenkachse des (ersten) bewegbaren Bauteils 30 relativ zu dem distalen Ende 23 des Schafts 30
- **40**: **zweites bewegbares Bauteil,** mit dem ersten bewegbaren Bauteil 30 gelenkig mechanisch verbunden
- 42: erste Stirnfläche des zweiten bewegbaren Bauteils 40
- 44: zweite Stirnfläche des zweiten bewegbaren Bauteils 40
- 46: Mantelfläche des zweiten bewegbaren Bauteils 40
- 47: Kontaktfläche an dem zweiten bewegbaren Bauteil 40, zu der Kontaktfläche 27 an dem Schaft 20 korrespondierend
- 48: Schwenkachse des zweiten bewegbaren Bauteils 40 relativ zu dem ersten bewegbaren Bauteil 30
- **50**: **erste optische Einrichtung** des Endoskops 10, an der Stirnfläche 24 an dem distalen Ende 23 des Schafts 30
- 52: Objektiv der ersten optischen Einrichtung 50
- 54: Bildsensor der ersten optischen Einrichtung 50
- 56: Lichteintrittsfläche der ersten optischen Einrichtung 50
- 58: optische Achse des Objektivs 52 der ersten optischen Einrichtung 50
- **60**: **zweite optische Einrichtung** des Endoskops 10, an der ersten Stirnfläche 32 oder an der Mantelfläche 36 des (ersten) bewegbaren Bauteils 30
- 62: Objektiv der zweiten optischen Einrichtung 60
- 64: Bildsensor der zweiten optischen Einrichtung 60
- 66: Lichteintrittsfläche der zweiten optischen Einrichtung 60
- 68: optische Achse des Objektivs 62 der zweiten optischen Einrichtung 60
- **70**: **dritte optische Einrichtung** des Endoskops 10, an der zweiten Stirnfläche 34 des (ersten) bewegbaren Bauteils 30 oder an der Mantelfläche 46 des zweiten bewegbaren Bauteils 40
- 72: Objektiv der dritten optischen Einrichtung 70
- 74: Bildsensor der dritten optischen Einrichtung 70
- 76: Lichtaustrittsfläche der dritten optischen Einrichtung 70
- 78: optische Achse des Objektivs 72 der dritten optischen Einrichtung 70
- 81: Sensor an dem (ersten) bewegbaren Bauteil 30
- 83: Welle, insbesondere mit zweitem Griffteil 18 verbunden
- 84: erste Seilscheibe an der Welle 83
- 85: zweite Seilscheibe an der Welle 83
- 86: erstes Zugseil, mit der ersten Seilscheibe 84 gekoppelt
- 87: zweites Zugseil, mit der zweiten Seilscheibe 85 gekoppelt
- 88: Zahnstange in dem Schaft 30, mit dem Zahnrad 89 kämmend
- 89: Zahnrad an dem bewegbaren Bauteil 40, mit der Zahnstange 88 kämmend
- 90: Außenschaft des Endoskops 10
- 92: von dem Außenschaft 90 mantelförmig umschlossener Hohlraum
- 94: elastisches Bauteil zwischen dem distalen Ende 23 des Schafts 20 und dem bewegbaren Bauteil 30

## Patentansprüche

1. **Endoskop** (10), mit:
einem **Schaft** (20) mit einer **Stirnfläche** (24) an einem distalen Ende (23) des Schafts (20), zum Einführen in einen zu betrachtenden Hohlraum;
einem **bewegbaren Bauteil** (30) mit einer Stirnfläche (32), das an dem distalen Ende (23) des Schafts (20) angeordnet und relativ zu dem distalen Ende (23) des Schafts (20) bewegbar ist zwischen einer ersten Konfiguration, in der der Schaft (20) in einen Hohlraum einführbar ist, und einer zweiten Konfiguration, die zur Betrachtung des Hohlraums vorgesehen ist;
einer **ersten optischen Einrichtung** (50) an der Stirnfläche (24) des Schafts (20),
einer **zweiten optischen Einrichtung** (60) an dem bewegbaren Bauteil (30),
wobei in der **ersten Konfiguration** die Stirnfläche (32) des bewegbaren Bauteils () gegenüber der Stirnfläche (24) des Schafts (20) angeordnet ist oder an dieser **anliegt,**
wobei in der **zweiten Konfiguration** das bewegbare Bauteil (30) **neben** dem distalen Ende (23) des Schafts (20) angeordnet ist, **dadurch gekennzeichnet, dass**
der Schaft (20) eine **erste Kontaktfläche** (27) aufweist,
das bewegbare Bauteil (30) eine zu der ersten Kontaktfläche (27) komplementär geformte **zweite Kontaktfläche** (37) aufweist,
in der ersten Konfiguration die zweite Kontaktfläche (37) von der ersten Kontaktfläche (27) beabstandet ist,
in der zweiten Konfiguration die zweite Kontaktfläche (37) die erste Kontaktfläche (27) flächig berührt.

2. Endoskop (10) nach dem vorangehenden Anspruch, bei dem
das bewegbare Bauteil (30) um eine vorbestimmte **Schwenkachse** (38) **orthogonal** zu einer Längsachse (28) des Schafts (20) schwenkbar ist,
die vorbestimmte Schwenkachse (38) **an der Stirnfläche** (24) **des Schafts** (20) angeordnet ist.

3. Endoskop (10) nach dem vorangehenden Anspruch, bei dem die **Schwenkachse** (38) **an dem Rand** der Stirnfläche (24) des Schafts (20) und **an dem Rand** der Stirnfläche (32) des bewegbaren Bauteils (30) angeordnet ist.

4. Endoskop (10) nach einem der vorangehenden Ansprüche, bei dem die **erste optische Einrichtung** (50) an der Stirnfläche (24) des Schafts (20) ein Objektiv (52) oder eine andere Abbildungseinrichtung zum Erzeugen eines reellen Bilds eines Objekts umfasst.

5. Endoskop (10) nach einem der vorangehenden Ansprüche, bei dem die **zweite optische Einrichtung** (60) **an der Stirnfläche** (32) des bewegbaren Bauteils (30) angeordnet ist.

6. Endoskop (10) nach einem der Ansprüche 1 bis 4, bei dem
das bewegbare Bauteil (30) im Wesentlichen die Gestalt eines Zylinders mit zwei Stirnflächen (32, 34) und einer Mantelfläche (36) aufweist,
die **zweite optische Einrichtung** (60) **an der Mantelfläche** (36) des bewegbaren Bauteils (30) angeordnet ist.

7. Endoskop (10) nach einem der vorangehenden Ansprüche in Rückbezug auf Anspruch 4, bei dem
die **zweite optische Einrichtung** (60) an dem bewegbaren Bauteil (30) ein **Objektiv** (62) oder eine andere Abbildungseinrichtung zum Erzeugen eines reellen Bilds eines Objekts umfasst,
die erste optische Einrichtung (50) und die zweite optische Einrichtung (60) zur Erfassung von **unterschiedlichen Wellenlängenbereichen** ausgebildet sind.

8. Endoskop (10) nach dem vorangehenden Anspruch in Rückbezug auf Anspruch 4, bei dem
die **zweite optische Einrichtung** (60) an dem bewegbaren Bauteil (30) ein **Objektiv** (62) oder eine andere Abbildungseinrichtung zum Erzeugen eines reellen Bilds eines Objekts umfasst,
eine **Winkelposition** des bewegbaren Bauteils (30) relativ zu dem distalen Ende (23) des Schafts (20) innerhalb eines vorbestimmten Winkelbereichs veränderbar ist,
um einen **Schnittpunkt** zwischen einer **optischen Achse** (58) der Abbildungseinrichtung (52) der ersten optischen Einrichtung (50) und einer optischen Achse (68) der Abbildungseinrichtung (62) der zweiten optischen Einrichtung (60) zu bewegen oder
um einen von der Abbildungseinrichtung (52) der ersten optischen Einrichtung (50) und der Abbildungseinrichtung (62) der zweiten optischen Einrichtung (60) **erfassten Raumwinkelbereich** zu vergrößern oder zu verkleinern.

9. Endoskop (10) nach einem der vorangehenden Ansprüche, ferner mit:
einer **dritten optischen Einrichtung** (70),
wobei die dritte optische Einrichtung (70) ein **Objektiv** (72) oder eine andere Einrichtung zum Erzeugen eines reellen Bilds eines Objekts umfasst.

10. Endoskop (10) nach dem vorangehenden Anspruch, ferner mit:
einem **weiteren bewegbaren Bauteil** (40), das mit dem bewegbaren Bauteil (30) gelenkig verbunden ist,
wobei die dritte optische Einrichtung (70) an dem weiteren bewegbaren Bauteil (40) angeordnet ist.

11. Endoskop (10) nach einem der vorhergehenden Ansprüche, ferner mit:
einer **Heatpipe** (21) oder einer anderen Wärmeleiteinrichtung in dem Schaft (20), die mit der ersten Kontaktfläche (27) unmittelbar wärmeleitfähig verbunden ist.

12. Endoskop (10) nach einem der vorangehenden Ansprüche, ferner mit:
einem **wärmeleitfähigen elastischen Bauteil** (94), das sowohl in der ersten Konfiguration als auch in der zweiten Konfiguration das bewegbare Bauteil (30) mit dem distalen Ende (23) des Schafts (20) wärmeleitfähig verbindet.

13. **Endoskop** (10) nach einem der vorhergehenden Ansprüche, mit:
einem **elastischen Bauteil** (94), das das bewegbare Bauteil (30) mit dem distalen Ende (23) des Schafts (20) mechanisch verbindet;
einem **Außenschaft** (90), der einen Hohlraum (92) umschließt,
wobei der Außenschaft (90) relativ zu dem Schaft (20) und dem bewegbaren Bauteil (30) bewegbar ist zwischen einer ersten Position, bei der der Schaft (20) und zumindest ein Teil des **bewegbaren Bauteils** (30) **in dem** von dem **Außenschaft** (90) umschlossenen Hohlraum (92) angeordnet sind, und einer zweiten Position, bei der das **bewegbare Bauteil** (30) **außerhalb des** von dem **Außenschaft** (90) umschlossenen Hohlraums (92) angeordnet ist,
wobei die in dem **elastischen Bauteil** (94) gespeicherte elastische Energie bei der zweiten Konfiguration minimal ist.

## Claims

1. Endoscope (10), having:
a shaft (20) with an end face (24) at a distal end (23) of the shaft (20), for insertion into a cavity that is to be viewed;
a movable component (30) with an end face (32), which component is arranged at the distal end (23) of the shaft (20) and is movable, relative to the distal end (23) of the shaft (20), between a first configuration, in which the shaft (20) is insertable into a cavity, and a second configuration, which is provided for viewing the cavity;
a first optical device (50) on the end face (24) of the shaft (20),
a second optical device (60) on the movable component (30),
wherein, in the first configuration, the end face (32) of the movable component is arranged opposite the end face (24) of the shaft (20) or bears against it,
wherein, in the second configuration, the movable component (30) is arranged alongside the distal end (23) of the shaft (20), **characterized in that** the shaft (20) has a first contact surface (27),
the movable component (30) has a second contact surface (37) which is shaped to complement the first contact surface (27),
in the first configuration the second contact surface (37) is spaced apart from the first contact surface (27),
in the second configuration the second contact surface (37) touches the first contact surface (27) in planar fashion.

2. Endoscope (10) according to the preceding claim, in which
the movable component (30) is pivotable about a predetermined pivot axis (38) orthogonal to a longitudinal axis (28) of the shaft (20),
the predetermined pivot axis (38) is arranged on the end face (24) of the shaft (20).

3. Endoscope (10) according to the preceding claim, in which the pivot axis (38) is arranged on the edge of the end face (24) of the shaft (20) and on the edge of the end face (32) of the movable component (30).

4. Endoscope (10) according to one of the preceding claims, in which the first optical device (50) on the end face (24) of the shaft (20) comprises a lens (52) or another imaging device for generating a real image of an object.

5. Endoscope (10) according to one of the preceding claims, in which the second optical device (60) is arranged on the end face (32) of the movable component (30) .

6. Endoscope (10) according to one of Claims 1 to 4, in which
the movable component (30) has substantially the shape of a cylinder with two end faces (32, 34) and a lateral surface (36),
the second optical device (60) is arranged on the lateral surface (36) of the movable component (30).

7. Endoscope (10) according to one of the preceding claims with reference back to Claim 4, in which
the second optical device (60) on the movable component (30) comprises a lens (62) or another imaging device for generating a real image of an object,
the first optical device (50) and the second optical device (60) are designed to detect different wavelength ranges.

8. Endoscope (10) according to the preceding claim with reference back to Claim 4, in which
the second optical device (60) on the movable component (30) comprises a lens (62) or another imaging device for generating a real image of an object,
an angular position of the movable component (30) relative to the distal end (23) of the shaft (20) can be changed within a predetermined angular range,
in order to move an intersection between an optical axis (58) of the imaging device (52) of the first optical device (50) and an optical axis (68) of the imaging device (62) of the second optical device (60) or
in order to enlarge or reduce a solid angle range covered by the imaging device (52) of the first optical device (50) and the imaging device (62) of the second optical device (60).

9. Endoscope (10) according to one of the preceding claims, further having:
a third optical device (70),
wherein the third optical device (70) comprises a lens (72) or another device for generating a real image of an object.

10. Endoscope (10) according to the preceding claim, further having:
a further movable component (40), which is connected to the movable component (30) in an articulated manner,
wherein the third optical device (70) is arranged on the further movable component (40).

11. Endoscope (10) according to one of the preceding claims, further having:
a heat pipe (21) or another heat conduction device, which is arranged in the shaft (20) and is connected directly to the first contact surface (27) in a thermally conductive manner.

12. Endoscope (10) according to one of the preceding claims, further having:
a thermally conductive elastic component (94) which connects the movable component (30) to the distal end (23) of the shaft (20) both in the first configuration and in the second configuration.

13. Endoscope (10) according to one of the preceding claims, having:
an elastic component (94), which mechanically connects the movable component (30) to the distal end (23) of the shaft (20);
an outer shaft (90), which encloses a cavity (92), wherein the outer shaft (90) is movable relative to the shaft (20) and to the movable component (30) between a first position, in which the shaft (20) and at least part of the movable component (30) are arranged in the cavity (92) enclosed by the outer shaft (90), and a second position, in which the movable component (30) is arranged outside the cavity (92) enclosed by the outer shaft (90),
wherein the elastic energy stored in the elastic component (94) is minimal in the second configuration.

## Revendications

1. Endoscope (10), comprenant :
une tige (20) pourvue d'une surface frontale (24) à une extrémité distale (23) de la tige (20) et destinée à être insérée dans une cavité à observer ;
un composant mobile (30) pourvu d'une surface frontale (32) et disposé à l'extrémité distale (23) de la tige (20) et mobile par rapport à l'extrémité distale (23) de la tige (20) entre une première configuration, dans laquelle la tige (20) peut être insérée dans une cavité, et une deuxième configuration permettant l'observation de la cavité ;
un premier dispositif optique (50) placé sur la surface frontale (24) de tige (20),
un deuxième dispositif optique (60) placé sur le composant mobile (30),
la surface frontale (32) du composant mobile () étant disposée, dans la première configuration, en face de la surface frontale (24) de la tige (20) ou en appui sur ladite surface frontale,
le composant mobile (30) étant disposé, dans la deuxième configuration, à côté de l'extrémité distale (23) de la tige (20), **caractérisé en ce que** la tige (20) comporte une première surface de contact (27),
le composant mobile (30) comporte une deuxième surface de contact (37) de forme complémentaire de celle de la première surface de contact (27),
la deuxième surface de contact (37) est espacée, dans la première configuration, de la première surface de contact (27),
la deuxième surface de contact (37) vient, dans la deuxième configuration, en contact surfacique avec la première surface de contact (27).

2. Endoscope (10) selon la revendication précédente, dans lequel
le composant mobile (30) peut pivoter sur un axe de pivotement prédéterminé (38) orthogonal à un axe longitudinal (28) de la tige (20),
l'axe de pivotement prédéterminé (38) est disposé sur la surface frontale (24) de la tige (20).

3. Endoscope (10) selon la revendication précédente, dans lequel l'axe de pivotement (38) est disposé au bord de la surface frontale (24) de la tige (20) et au bord de la surface frontale (32) du composant mobile (30) .

4. Endoscope (10) selon l'une des revendications précédentes, dans lequel le premier dispositif optique (50) placé sur la surface frontale (24) de la tige (20) comprend un objectif (52) ou un autre dispositif de reproduction destiné à générer une image réelle d'un objet.

5. Endoscope (10) selon l'une des revendications précédentes, dans lequel le deuxième dispositif optique (60) est disposé sur la surface frontale (32) du composant mobile (30).

6. Endoscope (10) selon l'une des revendications 1 à 4, dans lequel
le composant mobile (30) a sensiblement la forme d'un cylindre pourvu de deux surfaces frontales (32, 34) et d'une surface latérale (36),
le deuxième dispositif optique (60) est disposé sur la surface latérale (36) du composant mobile (30).

7. Endoscope (10) selon l'une des revendications précédentes par référence à la revendication 4, dans lequel
le deuxième dispositif optique (60) placé sur le composant mobile (30) comprend un objectif (62) ou un autre dispositif de reproduction destiné à générer une image réelle d'un objet,
le premier dispositif optique (50) et le deuxième dispositif optique (60) sont conçus pour détecter différentes gammes de longueurs d'onde.

8. Endoscope (10) selon la revendication précédente par référence à la revendication 4, dans lequel le deuxième dispositif optique (60) placé sur le composant mobile (30) comprend un objectif (62) ou un autre dispositif de reproduction destiné à générer une image réelle d'un objet,
une position angulaire du composant mobile (30) par rapport à l'extrémité distale (23) de la tige (20) peut être modifiée dans une plage angulaire prédéterminée, afin de déplacer un point d'intersection entre un axe optique (58) du dispositif de reproduction (52) du premier dispositif optique (50) et un axe optique (68) du dispositif de reproduction (62) du deuxième dispositif optique (60), ou
afin d'agrandir ou de réduire une plage d'angle solide détectée par le dispositif de reproduction (52) du premier dispositif optique (50) et du dispositif de reproduction (62) du deuxième dispositif optique (60).

9. Endoscope (10) selon l'une des revendications précédentes, comprenant en outre :
un troisième dispositif optique (70),
le troisième dispositif optique (70) comprenant un objectif (72) ou un autre dispositif destiné à générer une image réelle d'un objet.

10. Endoscope (10) selon la revendication précédente, comprenant en outre :
un autre composant mobile (40) qui est relié de manière articulée au composant mobile (30),
le troisième dispositif optique (70) étant disposé sur l'autre composant mobile (40).

11. Endoscope (10) selon l'une des revendications précédentes, comprenant en outre :
un caloduc (21) ou un autre dispositif thermoconducteur dans la tige (20) qui est directement relié de manière thermoconductrice à la première surface de contact (27) .

12. Endoscope (10) selon l'une des revendications précédentes, comprenant en outre :
un composant élastique thermoconducteur (94) qui relie de manière thermoconductrice le composant mobile (30) à l'extrémité distale (23) de la tige (20) dans les première et deuxième configurations.

13. Endoscope (10) selon l'une des revendications précédentes, comprenant :
un composant élastique (94) qui relie mécaniquement le composant mobile (30) à l'extrémité distale (23) de la tige (20) ;
une tige extérieure (90) qui renferme la cavité (92), la tige extérieure (90) étant mobile par rapport à la tige (20) et au composant mobile (30) entre une première position, dans laquelle la tige (20) et au moins une partie du composant mobile (30) sont disposées dans la cavité (92) renfermée par la tige extérieure (90), et une deuxième position dans laquelle le composant mobile (30) est disposé à l'extérieur de la cavité (92) renfermée par la tige extérieure (90), l'énergie élastique stockée dans le composant élastique (94) étant minimale dans la deuxième configuration.
